(19) Europäisches Patentamt
European Patent Office
Office européen des brevets

(11) **EP 4 660 178 A1**

(12) **EUROPEAN PATENT APPLICATION**
published in accordance with Art. 153(4) EPC

(43) Date of publication:
**10.12.2025 Bulletin 2025/50**

(21) Application number: **24750207.3**

(22) Date of filing: **29.01.2024**

(51) International Patent Classification (IPC):
*C07C 1/24* (2006.01)   *B01J 29/44* (2006.01)
*B01J 37/10* (2006.01)   *B01J 38/12* (2006.01)
*C07B 61/00* (2006.01)   *C07C 2/12* (2006.01)
*C07C 7/00* (2006.01)   *C07C 11/04* (2006.01)
*C07C 11/06* (2006.01)   *C07C 15/02* (2006.01)
*C07C 15/46* (2006.01)

(52) Cooperative Patent Classification (CPC):
**B01J 29/44; B01J 37/10; B01J 38/12; C07B 61/00;
C07C 1/24; C07C 2/12; C07C 7/00; C07C 11/04;
C07C 11/06; C07C 15/02; C07C 15/46;** Y02P 20/52

(86) International application number:
**PCT/JP2024/002643**

(87) International publication number:
**WO 2024/162258 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.01.2023 JP 2023011693**

(71) Applicant: **Asahi Kasei Kabushiki Kaisha
Tokyo 1000006 (JP)**

(72) Inventors:
• **URAYAMA, Teppei
Tokyo 100-0006 (JP)**

• **TSUJI, Yuki
Tokyo 100-0006 (JP)**
• **YAMAMOTO, Yuzuki
Tokyo 100-0006 (JP)**
• **KAWAMURA, Shogo
Tokyo 100-0006 (JP)**
• **MIYAZAKI, Ryusuke
Tokyo 100-0006 (JP)**
• **ONODERA, Kairi
Tokyo 100-0006 (JP)**

(74) Representative: **dompatent
Partnerschaft von
Patentanwälten und Rechtsanwälten mbB
Deichmannhaus am Dom
Bahnhofsvorplatz 1
50667 Köln (DE)**

(54) **METHOD FOR CONVERTING ETHANOL, ZEOLITE-CONTAINING CATALYST, METHOD FOR PRODUCING ZEOLITE-CONTAINING CATALYST, AND METHOD FOR PRODUCING HYDROCARBON, ETC**

(57) A method for converting ethanol, comprising a reaction step of feeding a mixed raw material containing ethanol into a reactor having a fixed bed filled with a zeolite-containing catalyst to obtain a reaction gas containing an olefin having 3 or more carbon atoms and water, wherein a zeolite contained in the zeolite-containing catalyst has an oxygen 10-membered ring structure, a molar ratio of Na/Al of the zeolite-containing catalyst is from 0.0050 to 0.25, and an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

EP 4 660 178 A1

FIG.1

## Description

### Technical Field

The present invention relates to a method for converting ethanol, a zeolite-containing catalyst, a method for producing a zeolite-containing catalyst, and a method for producing a hydrocarbon and the like.

### Background Art

Hydrocarbons such as lower olefins or aromatic compounds are core raw materials important for chemical industry. Particularly, various methods for producing propylene, which is anticipated to have growing demand, have been actively developed or modified. Among them, a method including contacting naphtha or olefins with a catalyst containing zeolite as an active species is known as a general method for producing propylene.

Production of chemical products such as lower olefins or aromatic compounds by using biomass-derived alcohols as raw materials, in addition to olefins, has received attention due to a rising demand for environmental protection in recent years. Particularly, ethanol is a compound whose production method from biomass raw materials has been established. Therefore, early development of an efficient method for converting ethanol has been expected.

For example, Patent Literatures 1 and 2 each disclose a method for producing a phosphorus-modified zeolite catalyst that accelerates conversion reaction from ethanol to propylene. Patent Literature 3 discloses a method for producing propylene, comprising producing propylene from an ethylene raw material containing water using a zeolite catalyst.

### Citation List

### Patent Literature

Patent Literature 1: Chinese Patent Publication No. 107649173A

Patent Literature 2: Japanese Patent No. 6229274

Patent Literature 3: Japanese Patent No. 5116043

### Summary of Invention

### Technical Problem

In an attempt to obtain hydrocarbons such as propylene or aromatic compounds by conversion of ethanol, water equimolar to raw material ethanol is produced as a by-product through dehydration reaction of ethanol. In general, water (water vapor) of 400°C or higher causes structural collapse associated with dealumination of a zeolite catalyst and reduces the catalyst performance of zeolite. In other words, in the case of converting ethanol by contacting a raw material containing ethanol with zeolite, water produced as a by-product reduces catalytic activity and thereby decreases the yield of a target compound as an operation time passes. Particularly, in the case of using a reactor having a fixed bed, deterioration of a catalyst leads to marked reduction in productivity because the catalyst is impossible to replace or additionally fill during reaction.

Patent Literatures 1 and 2 each disclose a method for producing a zeolite catalyst that converts an ethanol raw material to propylene, and show that catalytic activity is reduced as a reaction time passes. Patent Literature 3 discloses a method for converting an ethylene raw material containing water to propylene by contacting the ethylene raw material with a zeolite catalyst, and shows that propylene selectivity is reduced in several hours from the start of reaction.

Accordingly, an object of the present invention is to provide a method for converting ethanol by suppressing deterioration of catalyst performance ascribable to water vapor, and suppressing coke accumulation, a zeolite-containing catalyst, a method for producing a zeolite-containing catalyst, and a method for producing a hydrocarbon and the like.

### Solution to Problem

The present inventors have conducted diligent studies to attain the object described above, and consequently found that a target compound can be produced in stable yield by using a catalyst containing zeolite with an oxygen 10-membered ring that exhibits specific composition and physical properties, and thereby preventing structural collapse of zeolite even in ethanol conversion involving production of water as a by-product, and coke accumulation on a catalyst ascribable to side reaction, and suppressing reduction in catalytic activity.

Specifically, the present invention encompasses the following embodiments.

<1> A method for converting ethanol, comprising

a reaction step of feeding a mixed raw material containing ethanol into a reactor having a fixed bed filled with a zeolite-containing catalyst to obtain a reaction gas containing an olefin having 3 or more carbon atoms and water, wherein

a zeolite contained in the zeolite-containing catalyst has an oxygen 10-membered ring structure,

a molar ratio of Na/Al of the zeolite-containing catalyst is from 0.0050 to 0.25, and

an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

<2> The method for converting ethanol according to <1>, wherein the acid content Ac (unit: $\mu$mol/g) per weight of the zeolite-containing catalyst satisfies the following expression (1):

$$Ac \leq 25 - 60 \times [\text{molar ratio of Na/Al}] \ ... \ (1).$$

<3> The method for converting ethanol according to <1> or <2>, wherein the acid content per weight of the zeolite-containing catalyst is 0.5 $\mu$mol/g or more.

<4> The method for converting ethanol according to any of <1> to <3>, wherein an acid content per weight of the zeolite determined from an amount of ammonia desorbed at from 100 to 650°C in ammonia temperature-programmed desorption measurement of the zeolite-containing catalyst is 75 $\mu$mol/g or less.

<5> The method for converting ethanol according to any of <1> to <4>, wherein a content of sodium contained in the zeolite-containing catalyst is 100 ppm by mass or less.

<6> The method for converting ethanol according to any of <1> to <5>, wherein a mass ratio of Si/Al of the zeolite-containing catalyst is from 300 to 3000.

<7> The method for converting ethanol according to any of <1> to <6>, wherein a molar ratio of silica/alumina of the zeolite in the zeolite-containing catalyst is from 600 to 2000.

<8> The method for converting ethanol according to any of <1> to <7>, wherein the zeolite-containing catalyst comprises at least one dope element selected from the group consisting of phosphorus and an element of group 11.

<9> The method for converting ethanol according to <8>, wherein a content of the dope element is 2.0% by mass or less based on the total amount of the zeolite-containing catalyst.

<10> The method for converting ethanol according to any of <1> to <9>, wherein an aluminum content is from 0.01% by mass to 1% by mass based on the total amount of the zeolite-containing catalyst.

<11> The method for converting ethanol according to any of <1> to <10>, wherein the mixed raw material contains ethylene.

<12> The method for converting ethanol according to any of <1> to <11>, wherein an ethanol content of the mixed raw material is 30% by mass or more based on the mixed raw material.

<13> The method for converting ethanol according to any of <1> to <12>, wherein a molar ratio of ethylene/ethanol of the mixed raw material is from 0.20 to 2.50.

<14> The method for converting ethanol according to any of <1> to <13>, further comprising a regeneration step of contacting the zeolite-containing catalyst subjected to the reaction step with an oxygen-containing gas heated to 400°C or higher, and subjecting the zeolite-containing catalyst again to the reaction step.

<15> A method for producing propylene, comprising
a propylene-separating step of separating a fraction mainly comprising propylene from a reaction gas obtained by the method for converting ethanol according to any of <1> to <14>.

<16> A method for producing an aromatic compound, comprising
an aromatic compound-separating step of separating a fraction mainly comprising an aromatic compound from a reaction gas obtained by the method for converting ethanol according to any of <1> to <14>.

<17> A zeolite-containing catalyst, wherein

a zeolite contained in the zeolite-containing catalyst has an oxygen 10-membered ring structure,

a molar ratio of Na/Al of the zeolite-containing catalyst is from 0.0050 to 0.25, and

an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

<18> The zeolite-containing catalyst according to <17>, wherein a mass ratio of Si/Al of the zeolite-containing catalyst is from 300 to 3000.

<19> The zeolite-containing catalyst according to <17> or <18>, wherein the zeolite-containing catalyst is a catalyst for obtaining an olefin having 3 or more carbon atoms from a mixed raw material containing ethanol.

<20> The zeolite-containing catalyst according to any of <17> to <19>, wherein the zeolite-containing catalyst comprises at least one dope element selected from the group consisting of phosphorus and an element of group 11.

<21> The zeolite-containing catalyst according to <20>, wherein a content of the dope element is 2.0% by mass or less based on the total amount of the zeolite-containing catalyst.

<22> The zeolite-containing catalyst according to any of <1> to <21>, wherein an aluminum content is from 0.01% by mass to 1% by mass based on the total amount of the zeolite-containing catalyst.

<23> A method for producing a zeolite-containing catalyst, comprising a steaming step of contacting the zeolite-containing catalyst with water vapor at a steaming temperature of 450°C or higher, wherein

a zeolite contained in the zeolite-containing catalyst is zeolite having an oxygen 10-membered ring structure,

a molar ratio of Na/Al of the zeolite-containing catalyst is from 0.0050 to 0.25, and

an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

<24> A method for producing a hydrocarbon, comprising

a reaction step of feeding a raw material containing ethanol into a reactor having a fixed bed filled with a zeolite-containing catalyst to obtain a reaction gas containing an olefin having 3 or more carbon atoms and water, wherein

a zeolite contained in the zeolite-containing catalyst is zeolite having an oxygen 10-membered ring structure,

a molar ratio of Na/Al of the zeolite-containing catalyst is from 0.0050 to 0.25, and

an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

## Advantageous Effect of Invention

The present invention can provide a method for converting ethanol by suppressing deterioration of catalyst performance ascribable to water vapor, and suppressing coke accumulation, a zeolite-containing catalyst, a method for producing a zeolite-containing catalyst, and a method for producing a hydrocarbon and the like.

## Brief Description of Drawings

[Figure 1] Figure 1 shows a schematic view of one embodiment of a fixed-bed single-stage adiabatic reactor.

[Figure 2] Figure 2 shows a schematic view of one embodiment of an apparatus for converting ethanol.

[Figure 3] Figure 3 shows a schematic view of one embodiment of an apparatus for converting ethanol.

[Figure 4] Figure 4 shows reaction results of Example 1.

## Description of Embodiments

Hereinafter, the present invention will be specifically described. The present invention is not limited by the following embodiments (present embodiment) and can be carried out through various changes or modification without departing from the spirit of the present invention.

Herein, a numerical range represented by using "to" means a range including the numerical values described before and after "to" as the minimum value and the maximum value, respectively. In numerical ranges described in stages herein, the upper limit value or the lower limit value of a numerical range at a certain stage may be arbitrarily combined with the upper limit value or the lower limit value of a numerical range at a different stage.

[Method for converting ethanol]

The method for converting ethanol according to the present embodiment comprises

a reaction step of feeding a mixed raw material containing ethanol into a reactor having a fixed bed filled with a zeolite-containing catalyst to obtain a reaction gas containing an olefin having 3 or more carbon atoms and water, wherein

a zeolite contained in the zeolite-containing catalyst has an oxygen 10-membered ring structure (also simply referred to as a "10-membered ring structure"),

a molar ratio of Na/Al of the zeolite-containing catalyst is from 0.0050 to 0.25, and

an acid content per weight of the zeolite-containing catalyst determined from an amount of ammonia desorbed at from 100 to 650°C in ammonia temperature-programmed desorption measurement of the zeolite-containing catalyst (hereinafter, also referred to as a "TPD acid content per catalyst weight") is 75 $\mu$mol/g or less.

The present embodiment described above employs the zeolite-containing catalyst mentioned above and can thereby provide a method for converting ethanol by suppressing deterioration of catalyst performance ascribable to water vapor, and suppressing coke accumulation, a zeolite-containing catalyst, a method for producing a zeolite-containing catalyst, a method for producing a hydrocarbon, a method for producing propylene, and a method for producing an aromatic compound. In other words, the acidic nature of zeolite is controlled, and the affinity of the zeolite for water (water vapor) is reduced. This can presumably maintain the catalytic activity of the zeolite under reaction conditions where water vapor coexists therewith, and suppress reduction in activity of the zeolite-containing catalyst associated with a lapse of a reaction time. Furthermore, the amount of sodium in the zeolite-containing catalyst is controlled. This can presumably prevent structural collapse of zeolite and coke accumulation on a catalyst ascribable to side reaction, and suppress reduction in activity of the zeolite-containing catalyst under reaction conditions where ethanol exists. The zeolite-containing catalyst can also suppress deterioration of catalytic activity in a catalyst regeneration step involving generation of water vapor.

The present embodiment described above can convert ethanol to a target compound such as propylene or an aromatic compound in good yield. The present embodiment described above can further keep an ethylene conversion rate constant and can stably produce ethylene.

[Reactor]

In the present embodiment, the reaction step is carried out by filling a zeolite-containing catalyst into a reactor having a fixed bed (hereinafter, also referred to as a "fixed-bed reactor"). A reactor of any mode, i.e., an adiabatic reactor or an isothermal reactor, can be used as the fixed-bed reactor. Among them, a fixed-bed adiabatic reactor can be preferably used in view of excellent operability. A heating apparatus for heating the raw material can be provided, if necessary, upstream of the reactor.

(Fixed-bed adiabatic reactor)

For the fixed-bed adiabatic reactor, see the description of Adiabatic Fixed-Bed Reactors (Elsevier, 2014, Ch. 1, P. 4, L. 5-24 ISBN:978-0-12-801306-9). The fixed-bed adiabatic reactor is more preferably a fixed-bed single-stage adiabatic reactor, which has a single stage of a fixed catalyst bed. Since a carbonaceous material (coke) accumulates on a catalyst in association with reaction, a multi-column switchable fixed-bed single-stage adiabatic reactor, which is capable of removing this carbonaceous material by combustion while continuing the reaction, is preferred.

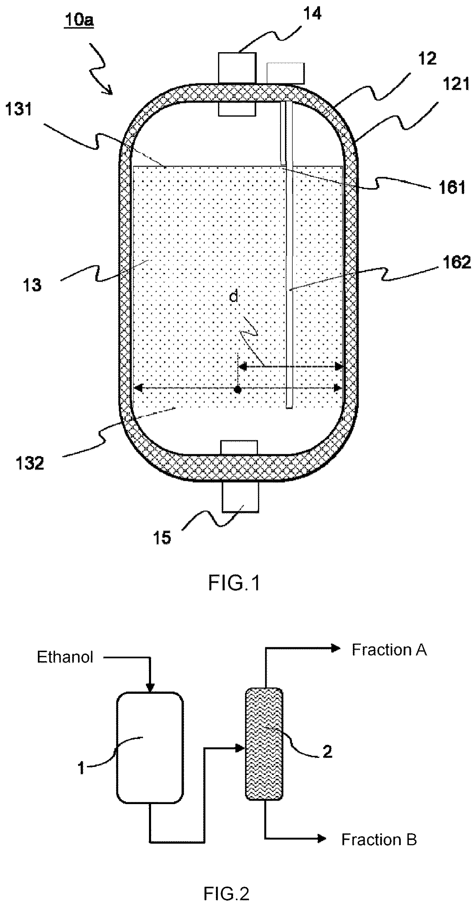

Figure 1 is a schematic view of the configuration of the fixed-bed single-stage adiabatic reactor. Fixed-bed single-stage adiabatic reactor 10a has reaction housing 12 having heat insulation material 121 provided on its outer periphery, catalyst bed 13, reactor inlet 14, and reactor outlet 15. The reaction housing 12, which has the heat insulation material 121 provided on its outer periphery, does not allow the internal heat of the reactor to escape to the outside. In the production method according to the present embodiment, the internal temperature of the reactor can be controlled through exothermic and endothermic reactions.

The catalyst bed 13 is filled with a catalyst mentioned later. First sheathed thermocouple 161 is disposed immediately upstream of catalyst bed inlet 131 of the catalyst bed 13. Second sheathed thermocouple 162 is disposed immediately downstream of catalyst bed outlet 132 of the catalyst bed 13. These thermocouples measure the temperatures of the mixed raw material immediately before contact with the catalyst bed inlet 131 and the reaction gas immediately after passage through the catalyst bed outlet 132. The positions of these thermocouples may be changed, if necessary. The catalyst bed 13 may be of multi-column type and is preferably of single-stage type as shown in Figure 1.

In the fixed-bed single-stage adiabatic reactor 10a, the raw material is introduced from the reactor inlet 14 and contacted with the catalyst bed 13, and the reaction gas is taken out of the reactor outlet 15.

[Zeolite-containing catalyst]

In the reaction step of the present embodiment, a zeolite-containing catalyst is used. The zeolite-containing catalyst contains zeolite. The zeolite-containing catalyst exhibits the ability to catalyze the conversion of ethanol and an olefin to a target compound such as propylene. A common challenge to conventional zeolite-based olefin production using an ethanol raw material includes deterioration of zeolite ascribable to water vapor. The method for converting ethanol according to the present embodiment controls the acidic nature of the zeolite-containing catalyst and reduces the affinity of the zeolite-containing catalyst for water (water vapor). This presumably facilitates maintaining the yield of a target compound over a long time.

The zeolite-containing catalyst may be a compact obtained by molding zeolite alone and is preferably a compact containing zeolite and a silica binder in view of excellent compressive strength.

In the conversion method of the present embodiment, the zeolite has an oxygen 10-membered ring structure. Examples of such zeolite include ZSM-5, ZSM-8, ZSM-11, ZSM-12, ZSM-21, ZSM-23, ZSM-35, and ZSM-38. Among them, MFI-type zeolite is preferred, and ZSM-5 is more preferred, in view of excellent catalyst performance (catalytic activity and durability against coking).

A method for synthesizing the zeolite of the present embodiment is not particularly limited, and the zeolite can be produced by optimizing various conditions for a conventionally known hydrothermal synthesis method of MFI-type zeolite. In general, an approach of efficiently obtaining MFI-type zeolite by a hydrothermal synthesis method includes a method including hydrothermally synthesizing the zeolite with an appropriate organic structure-directing agent (SDA), a method including hydrothermally synthesizing the zeolite by adding hydrothermally synthesized MFI zeolite as seed crystals, or a method including hydrothermally synthesizing the zeolite by adding a seed slurry at a crystalline stage. In this context, examples of the organic structure-directing agent used include ammonium salts, urea compounds, amine, and alcohols. Not only organic SDA but an inorganic cation or anion is known to be involved to direct the structure, and the zeolite synthesis depends on complex work of individual components. The hydrothermal synthesis method of MFI-type zeolite as mentioned above can produce a suitable catalyst by appropriately optimizing synthesis conditions such as the type of a raw material or an additive (SDA), the amount of the additive, pH, a silica/alumina molar ratio, a medium, raw material charging composition (e.g., the abundance ratio of a cation or an anion), a synthesis temperature, and a synthesis time.

Specific examples thereof include a synthesis method involving use of a seed slurry described in Japanese Patent No. 5426983, and methods illustrated in The Hydrothermal Synthesis of Zeolites (Chemical Reviews, 2003, 103, 663-702).

Commercially available zeolite may be used as long as the zeolite is the aforementioned MFI zeolite having specific physical properties and composition.

(TPD acid content per catalyst weight)

In the method for converting ethanol according to the present embodiment, a zeolite-containing catalyst is used in which the acid content per weight of the zeolite-containing catalyst determined from the amount of ammonia desorbed at 100 to 650°C in ammonia temperature-programmed desorption measurement of the zeolite-containing catalyst is 75 $\mu$mol/g or less. The catalyst having the TPD acid content in this range can suppress deterioration of catalyst performance ascribable to water vapor. The acid site of zeolite contributes to activity as a catalyst and however, also functions as an autocatalyst which promotes hydrolysis of the zeolite structure. Therefore, a catalyst having too large an acid content tends to facilitate progression of structural collapse of zeolite in a reactor where a reaction raw material contains ethanol and high-temperature water vapor coexists therewith. Hence, the TPD acid content controlled to the specific range is considered to lead to structural maintenance of the zeolite and functional maintenance as a catalyst. Too many active sites cause polymerization reaction to progress excessively so that coke easily accumulates on catalyst surface. Therefore, the acid content controlled to be equal to or less than the upper limit value leads to the amount of coke kept in a proper range. However, the factor is not limited thereto.

The TPD acid content per catalyst weight is preferably 40 $\mu$mol/g or less, more preferably 30 $\mu$mol/g or less, further preferably 25 $\mu$mol/g or less. The TPD acid content per catalyst weight is preferably 0.5 $\mu$mol/g or more, more preferably 1.5 $\mu$mol/g or more, further preferably 5.0 $\mu$mol/g or more, because of excellent catalytic activity per catalyst weight.

As a result of conducting experiments and studies, sodium heretofore used in acid content adjustment of a catalyst has been found to promote catalyst deterioration in the reaction step of the present embodiment. Thus, in the method for converting ethanol according to the present embodiment, the amount of sodium is preferably controlled, in addition to the acid content, and the TPD acid content Ac (unit: $\mu$mol/g) per catalyst weight preferably satisfies the following expression (1):

$$Ac \leq 25 - 60 \times [\text{molar ratio of Na/Al}] \ldots (1)$$

in view of higher improvement in durability of the catalyst. The molar ratio of Na/Al is the molar ratio between the amounts of sodium and aluminum contained in the zeolite-containing catalyst.

Sodium contained in the zeolite-containing catalyst exhibits strong basicity by coexistence with water or ethanol under conditions of the reaction step of the present embodiment, promotes structural collapse of zeolite having poor base resistance, and accelerates catalyst deterioration. Sodium also exhibits Lewis acidity at the same time with basicity and therefore promotes coke accumulation on a catalyst. Thus, for stably producing a target compound, it is necessary to control the amount of sodium, in addition to controlling the acid content of the zeolite-containing catalyst. In other words, only a smaller acid content is acceptable for a zeolite-containing catalyst rich in sodium. The relationship in which the upper limit value of a suitable acid content is decreased with increase in sodium level is represented by the expression (1).

The first term 25 in the right-hand side of the expression (1) represents a suitable acid content per weight of the catalyst because of excellent durability of the zeolite structure.

The second term in the right-hand side of the expression (1) represents the influence of sodium on the durability of the catalyst by multiplying the Na/Al molar ratio of the zeolite-containing catalyst by a coefficient 10. In the second term, not the amount of sodium based on the zeolite-containing catalyst, but the Na/Al molar ratio of the zeolite-containing catalyst, is used as a variable because a zeolite-containing catalyst having a larger amount of an acid site, i.e., a larger content of aluminum, can mitigate the adverse influence of sodium even though the amount of sodium based on the zeolite-containing catalyst is the same. The coefficient 60 has been determined from an example that exhibited suitable catalyst performance in experiments and studies.

Structural collapse caused by dealumination of zeolite ascribable to water vapor is attributed to the acidic nature of the zeolite. The reaction step can be carried out with dealumination suppressed by controlling the acidic nature of the zeolite-containing catalyst. However, the factor is not limited thereto.

For the zeolite-containing catalyst as described above, the acid content per weight of the zeolite determined from the amount of ammonia desorbed at from 100 to 650°C in ammonia temperature-programmed desorption measurement of the zeolite-containing catalyst (hereinafter, referred to as a "TPD acid content per zeolite weight") is preferably 75 µmol/g or less, more preferably 40 µmol/g or less, further preferably 30 µmol/g or less, because of excellent durability of the zeolite structure. The TPD acid content per zeolite weight is preferably 0.5 µmol/g or more, more preferably 1.5 µmol/g or more, further preferably 5.0 µmol/g or more.

The TPD acid content per catalyst weight can be calculated by use of a calibration curve method from a desorption spectrum obtained by adsorbing ammonia onto a degassed zeolite-containing catalyst by circulation of a diluted ammonia gas, followed by heating. The area value at from 100°C to 650°C of the spectrum obtained by the measurement is used for determining the TPD acid content. More specifically, this approach is based on a method described in Examples. The TPD acid content per zeolite weight is calculated by dividing the TPD acid content per zeolite-containing catalyst weight obtained by the method described above, by the weight ratio of the zeolite in the zeolite-containing catalyst. In this context, the TPD acid content means an (initial) acid content before the zeolite-containing catalyst is used in the reactor.

The acid content maintenance rate represented by the expression given below in the zeolite-containing catalyst is preferably 50% or more, more preferably 60% or more, further preferably 70% or more. The upper limit of the acid content maintenance rate is not particularly limited and may be 98% or less.

Acid content maintenance rate (%) = Acid content (µmol/g) after 6.0-hr STM treatment / Acid content (µmol/g) after 1.0-hr STM treatment $\times$ 100

The acid content after 6.0-hr STM treatment is the TPD acid content of the zeolite-containing catalyst treated with water vapor for 6 hours. The acid content after 1.0-hr STM treatment is the TPD acid content of the zeolite-containing catalyst treated with water vapor for 1 hour.

The water vapor treatment is performed by setting a helium gas flow rate to 50 mL/min, bubbling in water heated to 80°C, cooling to 75°C using a condenser, then providing a zeolite-containing catalyst heated to 650°C, and contacting water vapor with the zeolite-containing catalyst.

(Mesopore volume of zeolite-containing catalyst)

In the method for converting ethanol according to the present embodiment, the mesopore volume of the zeolite-containing catalyst used is preferably 0.15 cc/g or more, more preferably 0.20 cc/g or more, in view of excellent adsorption characteristics of the raw material. The mesopore volume can be measured by a mercury intrusion technique using a mercury porosimeter.

(Composition of zeolite-containing catalyst)

The silica/alumina ($SiO_2/Al_2O_3$) molar ratio of the zeolite contained in the zeolite-containing catalyst of the present embodiment can be appropriately selected and is preferably from 20 to 3000, more preferably from 200 to 2500, further preferably from 600 to 2000, in view of excellent durability of the catalyst. The silica/alumina molar ratio of zeolite can be measured by a known method and can be determined, for example, by completely dissolving the zeolite in an aqueous alkali solution, and analyzing the resulting solution by plasma emission spectroscopy or the like.

The mass ratio of silicon (Si) to aluminum (Al) (Si/Al mass ratio) contained in the zeolite-containing catalyst of the present embodiment can be appropriately selected and is preferably from 125 to 3000, more preferably from 300 to 3000, in view of excellent durability of the catalyst. The Si/Al mass ratio can be measured by a known method and can be determined, for example, by completely dissolving the zeolite-containing catalyst in an aqueous alkali solution, and analyzing the resulting solution by plasma emission spectroscopy or the like. The reaction step can be carried out with catalyst performance maintained even under hydrothermal conditions by controlling the Si/Al mass ratio of the zeolite-containing catalyst and thereby reducing the affinity of the zeolite-containing catalyst for water. However, the factor is not limited thereto. The Si/Al mass ratio is preferably 3000 or less, more preferably 2500 or less, further preferably 2000 or less, because of excellent catalytic activity. The Si/Al mass ratio is preferably 125 or more, more preferably 300 or more, further preferably 500 or more, because deterioration of catalyst performance ascribable to water vapor is suppressed.

The molar ratio of sodium to aluminum (molar ratio of Na/Al) contained in the zeolite-containing catalyst of the present embodiment is from 0.0050 to 0.25, preferably from 0.0050 to 0.20, more preferably from 0.005 to 0.15. When the molar ratio of Na/Al is 0.25 or less, deterioration of catalyst performance ascribable to water vapor is suppressed. When the molar ratio of Na/Al is 0.0050 or more, coke accumulation is suppressed. The molar ratio of Na/Al is preferably 0.20 or less, more preferably 0.15 or less, because of excellent durability against catalyst deterioration ascribable to higher-temperature water vapor. The molar ratio of Na/Al is preferably 0.0060 or more, more preferably 0.0070 or more, because of higher suppression of coke generation.

Sodium is used as a portion of a raw material in a zeolite synthesis process or a catalyst molding process.

Therefore, the molar ratio of Na/Al of the zeolite-containing catalyst is generally larger than 0.5. For adjusting sodium contained in the zeolite-containing catalyst to the range mentioned above, it is effective to wash the zeolite-containing catalyst using an acid (hereinafter, this operation is also referred to as "acid washing") to remove sodium. The type of the acid for use in the acid washing is not particularly limited, and the molar ratio of Na/Al can be adjusted by, for example, washing treatment using an aqueous nitric acid solution. The acid concentration of the washing solution for use in the acid washing is preferably from 0.010 to 10 N because of excellent washing efficiency. The acid concentration of the washing solution is more preferably from 0.01 to 2.0 N, further preferably from 0.05 to 1.5 N, because deactivation of zeolite associated with the acid washing is suppressed. The acid washing does not particularly require heating or cooling and is preferably carried out at from 10 to 90°C, more preferably at from 20 to 70°C, in view of excellent washing efficiency. The acid washing is carried out, for example, by dipping the zeolite-containing catalyst in the washing solution. The dipping time is preferably from 0.10 to 10 hours, more preferably from 0.50 to 6.0 hours, because of excellent washing efficiency. The weight of the washing solution for use in one acid wash is preferably 500 parts by mass or less, more preferably 250 parts by mass or less, based on 10 parts by mass of the zeolite because of easy liquid waste disposal. The acid washing can be carried out a plurality of times to enhance a sodium removal rate, and is preferably carried out only once in catalyst production because of easy liquid waste disposal.

In addition, cation exchange is effective as a method for removing sodium retained at a cation site. The molar ratio of Na/Al can be adjusted, for example, by decreasing a sodium content in the zeolite-containing catalyst by ion exchange treatment using an aqueous silver nitrate solution. Alternatively, the amount of sodium in the zeolite-containing catalyst may be adjusted by decreasing a sodium content in a raw material for use in zeolite synthesis by acid washing of the raw material.

Sodium contained in the zeolite-containing catalyst forms a base site under conditions where water or ethanol exists, and thereby causes structural collapse of zeolite or coke generation and promotes catalyst deterioration. Accordingly, for suppressing catalyst deterioration in the reaction step of the present embodiment, it is desirable to reduce a sodium level in the zeolite. Sodium is a component that is easily mixed into a zeolite synthesis or catalyst molding step, and needs to be particularly managed. Thus, the generation of a base site during reaction is suppressed by controlling the molar ratio of Na/Al. Reduction in activity of the zeolite-containing catalyst associated with a lapse of a reaction time can presumably be thereby suppressed under reaction conditions where water vapor coexists therewith.

The content of sodium contained in the zeolite-containing catalyst of the present embodiment is preferably 1,000 ppm by mass or less, more preferably 100 ppm by mass or less, further preferably 10 ppm by mass or less, based on the total amount of zeolite-containing catalyst. The content is preferably 100 ppm by mass or less in view of suppressing coke generation associated with side reaction. In the present embodiment, the content of sodium in the zeolite-containing catalyst refers to a value found by XRF analysis.

(Dope element in zeolite-containing catalyst)

The zeolite-containing catalyst according to the present embodiment can comprise at least one dope element selected from the group consisting of phosphorus and an element belonging to group 11 of the periodic table (hereinafter, these elements are also collectively referred to as a "dope element"). Among them, the zeolite-containing catalyst preferably contains phosphorus or silver.

Examples of the form of the phosphorus include phosphorus polymers (e.g., polyphosphoric acid), phosphorus oxides (e.g., $P_2O_5$), and compounds having phosphorus added to aluminum of zeolite. A plurality thereof may be contained. Phosphorus is effective for suppressing dealumination of zeolite when the zeolite contains aluminum. In the method according to the present embodiment, water may be produced in a reactor and contained in ethanol as the raw material. Therefore, a high-temperature water vapor atmosphere which causes dealumination is created in the reactor. The zeolite-containing catalyst containing phosphorus can suppress dealumination of zeolite and attain higher improvement in durability of the catalyst.

The element belonging to group 11 of the periodic table includes copper, silver, and gold. The contained element belonging to group 11 of the periodic table suppresses dealumination of zeolite and enhances durability of the catalyst. The element belonging to group 11 of the periodic table is preferably silver in view of excellent supporting efficiency.

The content of the dope element contained in the zeolite-containing catalyst may be 2.0% by mass or less and is preferably from 0.01 to 2.0% by mass, based on the total amount of the zeolite-containing catalyst. The content is more preferably from 0.05 to 1.0% by mass in view of higher suppression of dealumination.

In the present embodiment, the content of the dope element in the zeolite-containing catalyst refers to a value found using an X-ray fluorescence analyzer. The content of phosphorus, copper, silver, or gold can be measured using a commercially available X-ray fluorescence analyzer under usual conditions in accordance with its instruction manual. In the case of using, for example, trade name "RIX3000" manufactured by Rigaku Corp., measurement conditions can involve a tube voltage of 50 kV and a tube current of 50 mA using P-K$\alpha$ ray.

In the present embodiment, phosphoric acid and/or phosphate (hereinafter, also referred to as a "phosphorus raw material") may be used as a raw material for phosphorus contained in the zeolite-containing catalyst. The phosphorus raw material is more preferably phosphate, and the phosphate is more preferably a compound having a solubility of 1 g or more in 100 g of water at 25°C.

Examples of the phosphoric acid include phosphoric acid and pyrophosphoric acid. Examples of the phosphate include phosphoric acid ammonium salts such as ammonium phosphate, diammonium hydrogen phosphate, ammonium dihydrogen phosphate, and ammonium sodium hydrogen phosphate, potassium hydrogen phosphate, aluminum hydrogen phosphate, sodium phosphate, and potassium phosphate. Among them, a phosphoric acid ammonium salt having a relatively high solubility in water is preferred, and at least one member selected from the group consisting of ammonium phosphate, diammonium hydrogen phosphate, and ammonium dihydrogen phosphate is more preferred. These compounds may each be used singly or may be used in combination of two or more.

In the present embodiment, a nitric acid metal salt such as copper nitrate or silver nitrate may be used as a raw material for the element belonging to group 11 of the periodic table contained in the zeolite-containing catalyst. A zeolite-containing catalyst containing sodium as a counter cation is used in ion exchange with the nitric acid metal salt and sintered to obtain a zeolite-containing catalyst containing an element belonging to group 11 of the periodic table. The ion exchange of sodium serving as a counter cation in zeolite with the nitric acid metal salt can be carried out by dipping the zeolite or the zeolite-containing catalyst in an aqueous solution of the nitric acid metal salt, followed by washing with water. In this respect, the ion exchange rate can be improved by carrying out the dipping and the washing with water a plurality of times. As described above, sodium remaining in the catalyst impairs the deterioration resistance of zeolite. Therefore, the zeolite-containing catalyst containing the element belonging to group 11 of the periodic table is preferably prepared using a proton-type or ammonium-type zeolite-containing catalyst.

(Method for molding zeolite-containing catalyst)

The zeolite-containing catalyst of the present embodiment can be produced by using the aforementioned zeolite having specific physical properties and composition, and molding the zeolite, for example, as described below. The molding method is not particularly limited, and a general method can be used. Specific examples thereof include a method including compression-molding a catalytic component, a method including extrusion-molding a catalytic component, and a spray dry molding method optimal for a fluidized-bed reaction scheme.

A binder can be used in the molding. The binder is not particularly limited, and, for example, silica, alumina, and kaolin can each be used alone or can be used as a mixture. Commercially available products can be used as these binders. The mass ratio of zeolite/binder is preferably in the range of from 10/90 to 90/10, more preferably in the range of from 20/80 to 80/20. The conversion method of the present embodiment requires precisely controlling an acid site on the catalyst. Therefore, the catalyst preferably does not contain any compound that exhibits acidic nature, other than zeolite. However, a binder such as alumina or kaolin exhibits acidic nature. Thus, a binder that exhibits no acidic nature is preferably used. Among them, a silica binder is more preferably used in view of excellent coking resistance. The total content of the zeolite and the binder may be from 80% to 100% by mass or may be from 90% to 100% by mass, based on the total amount of the zeolite-containing catalyst. The aluminum content of the zeolite-containing catalyst is preferably from 0.01% by mass to 1% by mass, more preferably from 0.03% by mass to 0.5% by mass, further preferably from 0.05% by mass to 0.1% by mass, based on the total amount of the zeolite-containing catalyst.

(Pretreatment step of zeolite-containing catalyst)

In the method for converting ethanol according to the present embodiment, the zeolite-containing catalyst after being optionally molded may be subjected to a pretreatment step prior to contact with a raw material. The pretreatment step is preferably a steaming step of performing heat treatment at a temperature of 450°C or higher in the presence of water vapor. The pretreatment tends to more remarkably produce the effect of suppressing the deterioration of the catalyst or improving selectivity. In the case of the method described above, the treatment is preferably performed at a temperature of 450°C or higher and 900°C or lower in an atmosphere of, but not particularly limited to, a mixed gas of air or an inert gas (such as nitrogen) and steam (water vapor) circulated under conditions involving a water vapor partial pressure of 0.01 atm or more. The heat treatment temperature is more preferably a temperature of 500°C or higher and 700°C or lower. This pretreatment step can be performed using a reactor for converting ethanol and ethylene.

(Shape of zeolite-containing catalyst)

In the method for converting ethanol according to the present embodiment, a zeolite-containing catalyst having various shapes such as a cylindrical shape or a ring shape can be used, and a cylindrical zeolite-containing catalyst is preferably used in view of excellent ease of handling. Among them, a cylindrical compact having a diameter of 1.6 mm or larger and a length of from 0.1 to 10.0 cm is preferably used in view of excellent strength of the catalyst.

[Mixed raw material]

In the reaction step of the present embodiment, a mixed raw material containing ethanol is used. Ethanol is preferably derived from biomass in view of excellent environmental friendliness. The biomass refers to an organic resource other than a fossil resource originating from an animal or a plant. The term "derived from biomass" refers to a compound produced by using the biomass as a raw material.

The ethanol content of the mixed raw material is preferably 30% by mass or more, more preferably 40% by mass or more, further preferably 50% by mass or more, in view of excellent production efficiency of a target compound.

In the reaction step of the present embodiment, ethanol is first converted to ethylene. Ethanol is highly reactive as compared with an olefin having 4 or more carbon atoms, and a larger ethanol content in the mixed raw material improves production efficiency of a light olefin such as propylene or ethylene, which is a target compound. The high reactivity of ethanol is attributed to the accessibility of such a substrate to a reaction active site of zeolite, i.e., because ethanol or ethylene having 2 carbon atoms has a smaller molecular size than that of an olefin having 4 or more carbon atoms and is insusceptible to restrictions of steric hindrance inside the pores of zeolite. The high reactivity of ethanol is also attributed to the presence of a functional group containing a heteroatom in ethanol, i.e., intramolecular maldistribution of electrons.

A mixed raw material rich in ethylene can be expected to have similar effects. However, ethanol is preferably used to increase the ratio of a raw material having 2 carbon atoms in the mixed raw material, in view of stable operation of an apparatus. This is because the step of converting ethylene to a target compound is exothermic reaction and an excessive content of ethylene elevates the internal temperature of the reactor and promotes coking of the catalyst.

The mixed raw material can comprise ethylene in addition to ethanol in view of easy compositional change of the raw material. Ethylene produced by any of various production methods can be used. For example, ethylene obtained through thermal decomposition of naphtha and/or ethane, direct or oxidative dehydrogenation reaction of ethane, or dehydration reaction of ethanol can be used. Among them, the mixed raw material preferably comprises ethylene and water such as a gas obtained through dehydration reaction of ethanol. Water is produced as a by-product in the process of converting ethanol to ethylene. Therefore, ethylene generally contains water. Ethylene obtained by dehydration of biomass-derived ethanol is preferably used in view of excellent environmental friendliness. The molar ratio of ethylene/ethanol of the mixed raw material is preferably from 0.20 to 2.50, more preferably from 0.40 to 2.0, in view of excellent ease of reaction control.

The mixed raw material may further comprise a hydrocarbon having from 4 to 6 carbon atoms. Examples of the hydrocarbon having from 4 to 6 carbon atoms include olefins having from 4 to 6 carbon atoms and saturated hydrocarbons having from 4 to 6 carbon atoms. Among them, an olefin having from 4 to 6 carbon atoms can provide a target compound such as propylene by contact with the zeolite-containing catalyst, as in ethylene and ethanol. Examples of the hydrocarbon having from 4 to 6 carbon atoms include butene, pentene, hexene, butane, pentane, and hexane. The molar ratio of olefin having from 4 to 6 carbon atoms/ethylene in the mixed raw material is preferably 3.0 or less, more preferably 1.0 or less, further preferably from 0.14 to 1.0. The term "olefin" includes linear, branched, and cyclic olefins as well as cycloparaffin.

In the method for converting ethanol according to the present embodiment, the mixed raw material may further comprise an oxygenate having from 1 to 6 carbon atoms other than ethanol. The oxygenate having from 1 to 6 carbon atoms can provide a target compound such as propylene by contact with a catalyst, as in ethylene and ethanol. Examples of the oxygenate having from 1 to 6 carbon atoms other than ethanol include methanol, propanol, dimethyl ether, and diethyl ether. The molar ratio of oxygenate having from 1 to 6 carbon atoms in the mixed raw material is preferably 1.0 or less, more preferably 0.5 or less.

Ethylene, ethanol, an olefin having from 4 to 6 carbon atoms, and an oxygenate having from 1 to 6 carbon atoms other than ethanol are also collectively referred to as an "effective raw material".

The mixed raw material may comprise, in addition to the effective raw material described above, a saturated aliphatic hydrocarbon such as paraffin, an olefin having 7 or more carbon atoms, and an oxygenate having 7 or more carbon atoms. Such a saturated aliphatic hydrocarbon, an olefin having 7 or more carbon atoms, and an oxygenate having 7 or more carbon atoms are capable of being converted to a target compound by contact with a conversion catalyst, as in ethylene and ethanol, though their reactivity is lower than that of the effective raw material mentioned above.

The mixed raw material may comprise an inert gas such as nitrogen, in addition to the aforementioned raw material capable of being converted to propylene by the reaction step. In addition, the mixed raw material may comprise hydrogen or methane as a dilution gas. It is however preferred to not dilute with hydrogen. Although hydrogen may be used for suppressing the coking deterioration of a catalyst, hydrogen also causes hydrogenation reaction of produced propylene or the like, which has an adverse effect of reducing propylene purity (propylene / (propylene + propane)) [mol/mol]. The method of the present embodiment has a small coking deterioration rate of a catalyst and permits stable operation even if dilution with hydrogen is not performed. Therefore, it is preferred to not dilute with hydrogen.

The total content of ethylene and ethanol in the mixed raw material is preferably from 30 to 100% by mass, more preferably from 40 to 100% by mass, further preferably from 50 to 100% by mass, based on the total amount of the effective raw material. Here, as for ethanol, the mass thereof in terms of ethylene is used in the calculation.

The total content of olefins having from 4 to 6 carbon atoms in the mixed raw material is preferably 65% by mass or less, more preferably from 10 to 55% by mass, based on the total amount of the effective raw material.

The mixed raw material may comprise diethyl ether and preferably does not comprise diethyl ether. The content of diethyl ether in the effective raw material is preferably 10% by mass or less, more preferably 5% by mass or less, further preferably 1% by mass or less.

In the conversion method of the present embodiment, the mixed raw material can comprise water. Ethylene and ethanol contained in the raw material are produced by various production methods and therefore contain "water generated in the production process". In this context, the "water generated in the production process" refers to moisture that is generated in an ethylene and/or ethanol production process and remains unremoved. In the conversion method of the present embodiment, the mixed raw material can comprise water in addition to the "water generated in the production process". The water is effective for decreasing an olefin partial pressure, thereby suppressing coking deterioration and improving the yield of a lower olefin. On the other hand, preferably, the mixed raw material comprises no water in addition to the "water generated in the production process" in view of excellent production efficiency of a target compound per raw material flow rate.

[Reaction conditions]

The reaction temperature in the reaction step is preferably from 400 to 600°C, more preferably from 450 to 580°C, further preferably from 480 to 550°C, in view of excellent yield of a target compound.

In the case of using an adiabatic reactor, the reaction temperature is as follows. The catalyst bed inlet temperature is the temperature of the raw material immediately before the raw material fluid comes into contact with the catalyst bed filled into the adiabatic reactor. The catalyst bed outlet temperature is the temperature of the reaction gas immediately after the reaction gas passes through the catalyst bed. In this context, the temperatures of the raw material and the reaction gas refer to temperatures at from 0 d to 0.8 d, wherein d represents the distance from the center of the reactor to the inner wall surface of the reactor when the center of the reactor in a plane perpendicular to the flow direction of a fluid is defined as 0. The inlet-outlet average reaction temperature (hereinafter, also simply referred to as a "reaction temperature") is defined as a value calculated according to the expression: [Catalyst bed inlet temperature + Catalyst bed outlet temperature] / 2, wherein the catalyst bed inlet temperature and the catalyst bed outlet temperature are measured, as shown in Figure 1. In the case of using an adiabatic reactor, the catalyst bed inlet temperature is preferably from 480°C to 550°C, and the catalyst bed outlet temperature is preferably from 480°C to 550°C. The temperature difference between the catalyst bed outlet temperature and the catalyst bed inlet temperature is preferably from -80 K to 80 K, more preferably from -60 K to 60 K.

The reaction pressure in the reaction step is preferably from 0.01 to 3.0 MPaG, more preferably from 0.01 to 1.0 MPaG.

The feed rate of the effective raw material is preferably from 0.1 to 1000 hr$^{-1}$, more preferably from 0.1 to 100 hr$^{-1}$, further preferably from 0.5 to 50 hr$^{-1}$, in terms of the weight hourly space velocity (WHSV) of the zeolite-containing catalyst. In the reaction step, WHSV is calculated by using ethanol in terms of ethylene as shown in the expression given below. The mass flow rate of the effective raw material fed is preferably 1 kg/hr or more, more preferably 10 kg/hr or more, further preferably 1 t/hr or more, in view of being excellent in the productivity of a target compound.

$$\text{WHSV (hr}^{-1}) = \text{Flow rate of effective raw material fed (kg/hr) / Amount (kg) of catalyst}$$

Flow rate of effective raw material (kg/hr) = Ethylene flow rate (kg/hr) + Ethanol flow rate in terms of ethylene (kg/hr) + Flow rate (kg/hr) of olefin having from 4 to 6 carbon atoms + Flow rate (kg/hr) of oxygenate having from 1 to 6 carbon atoms other than ethanol Ethanol flow rate in terms of ethylene (kg/hr) = Ethanol flow rate (kg/hr) × Molecular weight (g/mol) of ethylene / Molecular weight (g/mol) of ethanol

[Regeneration step]

The method for converting ethanol according to the present embodiment may comprise a regeneration step of contacting the zeolite-containing catalyst subjected to the reaction step with an oxygen-containing gas heated to 400°C or higher, and subjecting the zeolite-containing catalyst again to the reaction step (hereinafter, also referred to as a "regeneration step"). When the zeolite-containing catalyst is used in reaction for a long period, coke accumulates on the catalyst, causing coking deterioration. The regeneration step can combust coke accumulating on the zeolite-containing catalyst. Examples of the oxygen-containing gas for use in the regeneration step include air and a mixed gas of air or oxygen and an inert gas. The oxygen concentration of the gas to be contacted is preferably from 0.1 to 2.0% by volume. The contact temperature with this gas is preferably equal to or higher than a temperature higher by 20°C (preferably a temperature higher by 30°C, more preferably a temperature higher by 40°C) than that of the reaction step. Specifically, the contact temperature may be from 400 to 700°C. In the regeneration step, use of the zeolite catalyst subjected to the reaction step generates water by involving water derived from water vapor generated in the reaction step or by combusting coke. Thus, in the regeneration step, the zeolite catalyst is contacted with water vapor under a temperature condition of 400°C or higher which promotes structural collapse of zeolite. However, as shown in the present embodiment, the control of the TPD acid content of the specific zeolite-containing catalyst can reduce the affinity of zeolite for water (water vapor) and suppress deterioration of catalytic activity. Any regeneration method may be adopted, including extra-reactor regeneration, which involves discharging the catalyst from the reactor and performing regeneration treatment outside the reactor, and intra-reactor regeneration, which involves performing regeneration treatment inside the reactor without discharging the catalyst from the reactor. Alternatively, reaction-regeneration switching operation may be performed by adopting a switchable reactor.

(Reaction-regeneration switching operation)

The reaction-regeneration switching operation is an operational procedure of performing the reaction step and the regeneration step at the same time using a double-column or multi-column switchable reactor. In the case of, for example, a triple-column switchable reactor, two columns are used in the reaction step while the remaining one column is used in the catalyst regeneration. Then, the reaction step of one of the columns used in the reaction step is terminated, and the catalyst regeneration is performed instead. The reaction step is performed in the one column used in the catalyst regeneration. As a result, the catalyst regeneration can be performed while production capacity derived from two columns is maintained. Such a reaction format, also called merry-go-round scheme, is preferred in view of production efficiency because of the absence of the need of discontinuing the production step for catalyst regeneration.

[Product: reaction gas comprising olefin having 3 or more carbon atoms]

In the conversion method according to the present embodiment, the raw material is contacted with the zeolite-containing catalyst to obtain a reaction gas comprising an olefin having 3 or more carbon atoms. The reaction gas may comprise ethylene. The reaction gas may comprise hydrogen, an aliphatic hydrocarbon having from 1 to 3 carbon atoms, an aliphatic hydrocarbon having from 4 to 6 carbon atoms, an aromatic compound, and a hydrocarbon having 9 or more carbon atoms.

In the conversion method according to the present embodiment, an aliphatic hydrocarbon having from 1 to 3 carbon atoms, an aliphatic hydrocarbon having from 4 to 6 carbon atoms, an aromatic compound, and a hydrocarbon having 9 or more carbon atoms are referred to as target compounds.

[Separation step]

In the conversion method according to the present embodiment, a target compound can be efficiently refined by establishing a separation step and carrying out various separation operations. Among them, the conversion method preferably comprises the step of separating the reaction gas obtained in the reaction step mentioned above into fraction A mainly comprising a hydrocarbon having from 2 to 3 carbon atoms and fraction B mainly comprising a hydrocarbon having from 4 to 6 carbon atoms by a separation apparatus. The target compound can be efficiently separated by such separation into each fraction. Examples of the separation apparatus for use in the separation step include distillation columns, quenching columns, and decanters.

The reaction gas obtained by the reaction step mentioned above or a fraction separated from the reaction gas (hereinafter, also referred to as a "connection fraction") may be introduced to the refining system of an ethylene plant, and a target compound such as ethylene, propylene, or an aromatic compound can be separated therefrom.

The method according to the present embodiment may be carried out by an apparatus having reactor 1 and first distillation column 2, as shown in Figure 2. The reaction gas obtained by the reaction step performed in the reactor 1 can be separated into fraction A mainly comprising a hydrocarbon having from 1 to 3 carbon atoms and fraction B mainly comprising a hydrocarbon having from 4 to 6 carbon atoms by the first distillation column 2. The separation of ethylene and propylene from the reaction gas is efficiently performed by separating ethylene from the fraction A by a distillation column (not shown) and further separating propylene by a distillation column (not shown). Although not shown, at least a portion of the reaction gas and/or the fraction A may be introduced to the refining system of an ethylene plant, and a target compound such as ethylene or propylene can be separated from the reaction gas by the refining system.

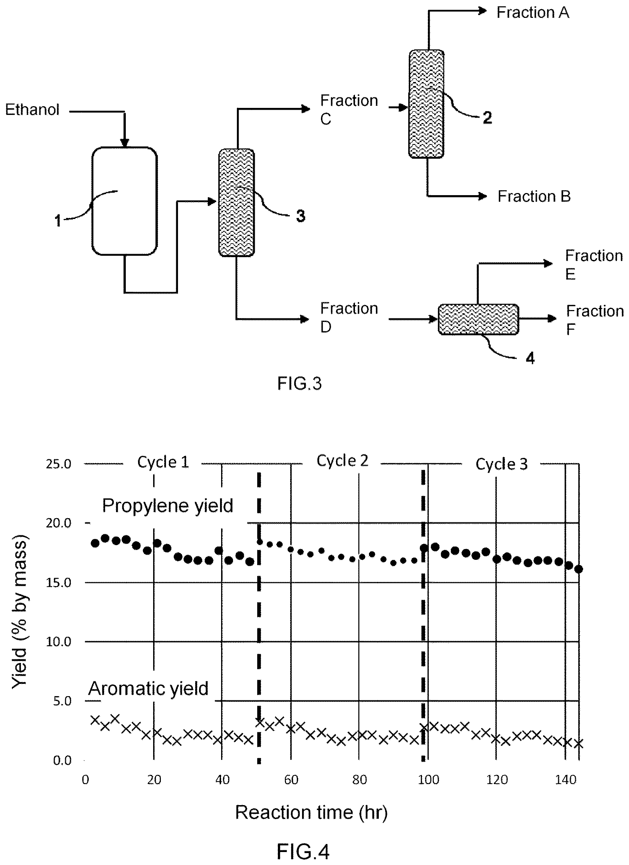

The method according to the present embodiment can be carried out by an apparatus having reactor 1, cooler 3, oil-water separator 4, and first distillation column 2, as shown in Figure 3. The reaction gas obtained by the reaction step performed in the reactor 1 is separated into fraction C mainly comprising a hydrocarbon having from 1 to 6 carbon atoms and fraction D mainly comprising water, a hydrocarbon having 7 or more carbon atoms, and an aromatic compound by the cooler 3. The phrase "mainly comprising water, a hydrocarbon having 7 or more carbon atoms, and an aromatic compound" in the fraction D means that the total amount of the water, the hydrocarbon having 7 or more carbon atoms, and the aromatic compound exceeds 50% by mass of the total amount of the fraction D. The fraction C is separated into fraction A mainly comprising a hydrocarbon having from 1 to 3 carbon atoms and fraction B mainly comprising a hydrocarbon having from 4 to 6 carbon atoms by the first distillation column 2. The fraction D is separated into fraction E mainly comprising a hydrocarbon having 7 or more carbon atoms and an aromatic compound and fraction F mainly comprising water by the oil-water separator 4. Refining of an aromatic compound can be efficiently carried out by introducing the fraction E to a refining system. Thus, refining of a target compound such as propylene or an aromatic compound can be efficiently carried out by separating the reaction gas into fraction A, fraction B, and fraction E. At least a portion of the fraction A, the fraction B, and the fraction E may be introduced to the refining system of an ethylene plant, and a target compound such as ethylene, propylene, or an aromatic compound can be separated from the reaction gas by the refining system.

The term "mainly comprising" for various fractions means that the total mass of the component described with the term "mainly comprising" exceeds 50% by mass based on each fraction. These separation steps can be carried out by combining various known methods such as distillation and extraction.

As described above, various hydrocarbons can be obtained by the method for converting ethanol according to the present embodiment. In short, the method for converting ethanol according to the present embodiment is a method for producing a hydrocarbon from another aspect. The hydrocarbon may be any of saturated hydrocarbons and unsaturated hydrocarbons. Examples of the unsaturated hydrocarbon include olefins and aromatic hydrocarbon compounds.

[Methods for producing hydrocarbon, etc.]

Various chemical products are obtained by separating target compounds from the reaction gases obtained by the present embodiment.

In short, the method for producing a hydrocarbon according to the present embodiment comprises contacting a mixed raw material containing ethylene and ethanol with a catalyst in an adiabatic reactor to obtain a reaction gas containing an olefin having 3 or more carbon atoms. The details of the production method are as described for the aforementioned method for converting ethanol, and its preferred embodiments are the same as therein.

Examples of the hydrocarbon obtained by the production method include: aliphatic unsaturated hydrocarbons such as olefins such as propylene, ethylene, butene, pentene, hexene, and heptene, and dienes such as 1,3-butadiene and isoprene; aromatic hydrocarbons such as benzene and toluene; and aliphatic saturated hydrocarbons such as ethane, propane, butane, and pentane. The aromatic hydrocarbons preferably have a boiling point of 500°C or lower at normal pressure.

A target hydrocarbon compound can be efficiently refined by introducing the obtained hydrocarbon to a refining system of a cracker.

The method for producing a hydrocarbon according to the present embodiment comprises:

a cracking step of decomposing a hydrocarbon having 2 or more carbon atoms; and
a refining step of refining a component obtained in the cracking step, wherein
in the refining step, a reaction gas obtained by the aforementioned method for converting ethanol or a refined fraction thereof is combined with the component.
Owing to the constitutional features described above, a target hydrocarbon can be obtained from an ethanol resource using a conventional cracker. For example, a bio-based hydrocarbon can be produced by using bioethanol as a raw material in the method for converting ethanol.

Examples of the hydrocarbon having 2 or more carbon atoms include ethylene, propylene, butene, paraffin, and aromatic hydrocarbons. Naphtha may be used as the hydrocarbon having 2 or more carbon atoms.

A pyrolytic furnace for use in an ethane cracker, a naphtha cracker, or the like may be used as the cracker.

In the refining step, a refining system for use in a conventional ethane cracker or naphtha cracker may be used, and distillation may be performed by a facility equipped with, for example, a distillation column and a quenching column.

The reaction gas obtained by the aforementioned method for converting ethanol or a refined fraction thereof is combined with the component in the refining step mentioned above. The location of combination is appropriately selected depending on the component to be combined therewith.

The method for producing a monomer according to the present embodiment comprises
an unsaturated hydrocarbon-separating step of separating a fraction mainly comprising an unsaturated hydrocarbon from a reaction gas obtained by the aforementioned method for converting ethanol.

Examples of the unsaturated hydrocarbon include aliphatic unsaturated hydrocarbons such as olefins such as propylene, ethylene, butene, butane, pentene, hexene, and heptene, and dienes such as 1,3-butadiene and isoprene.

The method for producing an olefin according to the present embodiment comprises
an olefin-separating step of separating a fraction mainly comprising an olefin from a reaction gas obtained by the aforementioned method for converting ethanol.

The method for producing propylene according to the present embodiment comprises
a propylene-separating step of separating a fraction mainly comprising propylene from a reaction gas obtained by the aforementioned method for converting ethanol.

The method for producing ethylene according to the present embodiment comprises
an ethylene-separating step of separating a fraction mainly comprising ethylene from a reaction gas obtained by the aforementioned method for converting ethanol.

The method for producing a diene according to the present embodiment comprises
a diene-separating step of separating a fraction mainly comprising a diene from a reaction gas obtained by the aforementioned method for converting ethanol.

The method for producing a monomer according to the present embodiment may further comprise the step of converting the unsaturated hydrocarbon.

The method for producing an acrylic monomer according to the present embodiment comprises:

an unsaturated hydrocarbon-separating step of separating a fraction mainly comprising an unsaturated hydrocarbon from a reaction gas obtained by the aforementioned method for converting ethanol; and

an acrylic monomer-producing step of obtaining an acrylic monomer from the unsaturated hydrocarbon obtained by the unsaturated hydrocarbon-separating step.

The acrylic monomer-producing step employs a known method for introducing the acrylic monomer from the unsaturated hydrocarbon.

The method for producing acrylonitrile according to the present embodiment comprises:

a propylene-separating step of separating a fraction mainly comprising propylene from a reaction gas obtained by the aforementioned method for converting ethanol; and

an acrylonitrile-producing step of obtaining acrylonitrile from the propylene obtained by the propylene-separating step.

The acrylic monomer-producing step employs a known method for introducing the acrylic monomer from the unsaturated hydrocarbon.

The method for producing styrene according to the present embodiment comprises:

an ethylene-separating step of separating a fraction mainly comprising ethylene from a reaction gas obtained by the aforementioned method for converting ethanol; and

a styrene-producing step of obtaining styrene from the ethylene obtained by the ethylene-separating step.

The monomers obtained by the production methods mentioned above may be further polymerized.

The method for producing a polymer according to the present embodiment comprises the step of polymerizing a monomer obtained by the aforementioned method for producing a monomer.

In the step of polymerizing a monomer, the polymerization can be performed by use of a conventional polymerization method and can be performed with various initiators and polymerization catalysts.

The method for producing an olefin-based polymer according to the present embodiment comprises the step of polymerizing a polymerizable composition comprising an olefin obtained by the aforementioned method for producing an olefin.

In this context, the polymerizable composition may be an olefin alone as a monomer or may contain an additional monomer having an unsaturated bond.

The method for producing a polypropylene-based polymer according to the present embodiment comprises the step of

polymerizing a polymerizable composition comprising propylene obtained by the aforementioned method for producing propylene.

In this context, the polymerizable composition may be propylene alone as a monomer or may contain an additional monomer having an unsaturated bond.

The method for producing a polyethylene-based polymer according to the present embodiment comprises the step of

polymerizing a polymerizable composition comprising ethylene obtained by the aforementioned method for producing ethylene.

In this context, the polymerizable composition may be ethylene alone as a monomer or may contain an additional monomer having an unsaturated bond.

The method for producing a diene-based polymer according to the present embodiment comprises the step of polymerizing a polymerizable composition comprising a diene obtained by the aforementioned method for producing a diene.

In this context, the polymerizable composition may be a diene alone as a monomer or may contain an additional monomer having an unsaturated bond.

The method for producing an acrylic monomer-based polymer according to the present embodiment comprises the step of

polymerizing a polymerizable composition comprising an acrylic monomer obtained by the aforementioned method for producing an acrylic monomer.

In this context, the polymerizable composition may be an acrylic monomer alone as a monomer or may contain an additional monomer having an unsaturated bond.

The method for producing an acrylonitrile-based polymer according to the present embodiment comprises the step of

polymerizing a polymerizable composition comprising acrylonitrile obtained by the aforementioned method for producing acrylonitrile.

In this context, the polymerizable composition may be acrylonitrile alone as a monomer or may contain an additional monomer having an unsaturated bond.

The method for producing a styrene-based polymer according to the present embodiment comprises the step of polymerizing a polymerizable composition comprising styrene obtained by the aforementioned method for producing styrene.

In this context, the polymerizable composition may be styrene alone as a monomer or may contain an additional monomer having an unsaturated bond.

An aromatic compound may be separated from the reaction gas obtained by the aforementioned method for converting ethanol.

The method for producing an aromatic compound according to the present embodiment comprises an aromatic compound separation step of separating a fraction mainly comprising an aromatic compound from a reaction gas obtained by the aforementioned method for converting ethanol.

Examples of the aromatic hydrocarbon include benzene, toluene, and xylene.

Examples

Hereinafter, the present embodiment will be described further specifically with reference to Examples. However, the present embodiment is not limited by the following Examples.

[Methods for measuring physical properties of catalyst]

Various physical properties of a catalyst were measured as described below.

(1) Molar ratio of silica/alumina of zeolite

Zeolite was completely dissolved in a sodium hydroxide solution to provide a solution. The amounts of Si and Al contained in the solution were measured by an ordinary method using an ICP (inductively coupled plasma) spectrometry apparatus (manufactured by Rigaku Corp., trade name "JY138"), and the molar ratio of silica/alumina was determined from the results. The measurement conditions were set to high-frequency power: 1 kw, plasma gas: 13 L/min, sheath gas: 0.15 L/min, nebulizer gas: 0.25 L/min, Si measurement wavelength: 251.60 nm, and Al measurement wavelength: 396.152 nm.

(2) Content of Al and content of Si in zeolite-containing catalyst

A zeolite-containing catalyst was completely dissolved in a sodium hydroxide solution to provide a solution. The amount of aluminum (Al) in the zeolite-containing catalyst was measured and determined by the same method as in (1).

(3) Content of dope element in zeolite-containing catalyst

The content of a dope element in a zeolite-containing catalyst was measured by a routine method using an X-ray fluorescence analyzer (manufactured by Rigaku Corp., trade name "RIX3000").

(4) Structural type of zeolite

The structural type of zeolite in a zeolite-containing catalyst was identified by measuring the X-ray diffraction pattern of the zeolite using an X-ray analysis apparatus (manufactured by Bruker, trade name "D8 Advance"), and referring to the diffraction pattern of known zeolite. The measurement conditions are as follows.

Cu negative electrode

Tube voltage: 40 kV

Tube current: 40 mA

Scan speed: 6 deg/min

(5) TPD acid content per zeolite-containing catalyst weight and TPD acid content per zeolite weight

The TPD acid content "acid content determined from the amount of ammonia desorbed at from 100 to 650°C in ammonia temperature-programmed desorption measurement" of a zeolite-containing catalyst was measured by the following method using temperature-programmed desorption spectroscopy apparatus BELCAT II.
In pretreatment, 1.0 g of a catalyst sample was heated to 650°C in He gas and cooled to 100°C. Subsequently, 5 vol% of ammonia gas diluted with helium gas was circulated in the catalyst sample in a state heated to 100°C so that ammonia was adsorbed to the catalyst sample. Then, helium gas containing saturated water vapor was circulated at 80°C in the catalyst sample in a state heated to 100°C to remove the ammonia physically adsorbed to the catalyst. Helium gas was circulated at 50 mL/min, and the sample was held at 100°C for 90 minutes. Then, the amount of ammonia desorbed was measured by heating from 100°C to 850°C under a heating rate condition of 5°C/min. The desorbed ammonia was detected using a mass spectrum at m/z = 16. An area value from a point at which the heating from 100°C toward 850°C was started to a point at which the temperature reached 650°C was calculated in the mass spectrum obtained by the measurement. The TPD acid content per zeolite-containing catalyst weight was calculated by use of the calibration curve method. The TPD acid content per zeolite weight was calculated from the obtained TPD acid content per zeolite-containing catalyst weight and the weight ratio of zeolite.

(Acid content maintenance rate)

In this Example, a zeolite-containing catalyst was treated with water vapor (STM treatment), and the acid content maintenance rate was calculated by observing an acid content over time. The water vapor treatment in the calculation of the acid content maintenance rate was carried out by setting a helium gas flow rate to 50 mL/min, bubbling in water heated to 80°C, cooling to 75°C using a condenser, and then contacting water vapor with a zeolite-containing catalyst heated to 650°C.
The acid content maintenance rate, which indicates the deterioration resistance of the zeolite-containing catalyst under hydrothermal conditions, was calculated according to the following expression.

Acid content maintenance rate (%) = Acid content ($\mu$mol/g) after 6.0-hr STM treatment / Acid content ($\mu$mol/g) after 1.0-hr STM treatment $\times$ 100

(6) Mesopore volume of zeolite-containing catalyst

The mesopore volume of a zeolite-containing catalyst was measured by the following method using a mercury intrusion porosimeter (AutoPore 9500 model manufactured by MicroMeritics Instrument Corp.).

In pretreatment, the zeolite-containing catalyst was first coarsely crushed and sifted through a sieve for particle size adjustment. The sample after particle size adjustment was dried. Then, 0.5 g of the sample was introduced to a measurement cell into which mercury was then filled, followed by pressurization. The mesopore volume was calculated from the obtained amount of mercury intruding.

(7) Content of sodium in zeolite-containing catalyst

The content of sodium in a zeolite-containing catalyst was measured by a routine method using an X-ray fluorescence analyzer (manufactured by Rigaku Corp., trade name "RIX3000") (hereinafter, this measurement is also simply referred to as "XRF analysis").

(Molar ratio of Na/Al)

The molar ratio of Na/Al was calculated according to the following expression.

Molar ratio of Na/Al of zeolite-containing catalyst = [Content (% by mass) of sodium / Atomic weight of sodium] / [(Content (% by mass) of aluminum / Atomic weight of aluminum]

(8) Strength of zeolite-containing catalyst

The strength of a zeolite-containing catalyst was calculated using a digital hardness tester (manufactured by Fujiwara Scientific Co., Ltd., trade name "KHT-40"). A catalyst sample dried at 120°C for 3 hours or longer was provided, and its compressive strength in the diameter direction was obtained (unit: N) in the hardness tester with an indenter of 3 mm in diameter attached thereto. A value (unit: N/mm) obtained by measuring 30 samples and dividing an average value by 3 was regarded as the strength of the zeolite-containing catalyst.

(9) Amount of coke

The amount of coke was calculated by the following method. A zeolite-containing catalyst after the completion of reaction was ground, and the rate of decrease in weight was measured in a temperature range from 500°C to 700°C using a thermogravimetric differential thermal analyzer. A value (unit: ppm by mass) obtained by dividing the rate of decrease in weight by the mass of an effective raw material fed was regarded as the amount of coke.

Mass (kg) of effective raw material fed = Mass flow rate of effective raw material fed (kg/hr) $\times$ Time (hr) from start to completion of reaction

[Analysis condition of thermogravimetric differential thermal analyzer]

Apparatus: TG-DTA8122 manufactured by Rigaku Corp.

Sample container: Pt pan ($\phi$5 mm $\times$ 2.5 mmh)

Reference sample: $\alpha$-Alumina

Atmosphere: Air (500 cc/min)

Temperature:

(1) Heated from room temperature to 120°C at 20°C/min

(2) Held at 120°C for 10 minutes

(3) Heated from 120°C to 900°C at 10°C/min

[Method for converting ethanol]

(Reactor)

In Examples and Comparative Examples given below, evaluation was conducted using the fixed-bed single-stage adiabatic reactor 1 shown in Figure 1.

(Temperature measurement)

The temperature of a catalyst bed inlet and the temperature of a catalyst bed outlet were measured by a thermocouple inserted from the outside of the reactor. Specifically, as shown in Figure 1, temperatures at from 0.5 d to 0.6 d were measured, wherein d represents the distance from the center of the reactor to the inner wall surface of the reactor when the center of the reactor in a plane perpendicular to the flow direction of a fluid was defined as 0. The influence of heat dissipation ascribable to the insertion of this thermocouple is negligibly small. The thermocouples were moved, if necessary, in the flow direction of a fluid, and the lowest internal temperature of the reactor was measured.

(Raw material)

Examples given below were carried out using a raw material containing ethanol. The raw material can comprise ethylene, 1-butene, or water in addition to ethanol. The molar ratio of ethylene/ethanol and the molar ratio of olefin having from 4 to 6 carbon atoms/ethylene in the raw material were calculated according to the following expressions.

Molar ratio (-) of ethylene/ethanol = Molar flow rate (mol/hr) of ethylene / Molar flow rate (mol/hr) of ethanol Molar ratio (-) of olefin having from 4 to 6 carbon atoms/ethylene = Flow rate (mol/hr) of olefin having from 4 to 6 carbon atoms / Molar flow rate (mol/hr) of ethylene (Evaluation of reaction in reaction step)

Reaction was carried out in accordance with Examples and Comparative Examples given below such that an inlet-outlet average reaction temperature was 530°C or 500°C. A portion of a gas from the reactor outlet was sampled every 3 hours from the start of the reaction, introduced to a gas chromatograph (TCD (Thermal Conductivity Detector) and FID (Flame Ionization Detector) detectors), and analyzed for reaction gas composition. The reaction was terminated 48 hours after the start of the reaction. An average value of GC analysis results from the start of the reaction to the termination of the reaction was calculated. The inlet-outlet average reaction temperature was calculated according to the following expression.

Inlet-outlet average reaction temperature (°C) = [Catalyst bed inlet temperature (°C) + Catalyst bed outlet temperature (°C)] / 2

[Analysis conditions for gas chromatograph]

(Analysis of reaction gas)

Apparatus: GC-2030 manufactured by Shimadzu Corp.

Column: Custom capillary column SPB-1 (inside diameter: 0.25 mm, length: 60 m, film thickness: 3.0 $\mu$m) manufactured by Supelco, Inc. (USA)

Amount of sample gas: 1 mL (a sampling line was kept at from 200°C to 300°C)

Heating program: Held at 40°C for 12 minutes, subsequently heated to 200°C at 5°C/min, and then held at 200°C for 22 minutes.

Split ratio: 200:1

Carrier gas (nitrogen) flow rate: 120 mL/min

FID detector: Air feed pressure of 50 kPa (approximately 500 mL/min), hydrogen feed pressure of 60 kPa (approximately 50 mL/min)

Measurement method: A TCD detector and an FID detector were connected in series. Compositional analysis was conducted on the basis of data on hydrogen detected in the TCD detector and data on oxygenates, such as hydrocarbons and ethanol, detected in the FID detector. The concentration of each component in a reaction gas was determined by use of the calibration curve method, and the mass per hour of the compound produced through reaction was determined.

(Ethylene yield)

The ethylene yield indicates selectivity for ethylene in the reaction step and was calculated according to the following expression.

Ethylene yield (% by mass) = Mass per hour of ethylene produced in reaction step (kg/hr) / Mass flow rate of effective raw material fed (kg/hr) $\times$ 100

(Ethylene conversion rate)

The raw material of the present embodiment does not necessarily comprise ethylene. Ethylene is produced in the reaction step of the present embodiment, and the smallest olefin is ethylene. Therefore, an ethylene conversion rate determined from the mass per hour of ethylene in a reaction gas was adopted as an index for evaluating catalytic activity. The ethylene conversion rate was calculated according to the following expression.

Ethylene conversion rate (% by mass) = (Mass flow rate of effective raw material fed (kg/hr) - Mass per hour of ethylene in reaction gas (kg/hr)) / Mass flow rate of effective raw material fed (kg/hr) $\times$ 100

Mass flow rate of effective raw material fed (kg/hr) = Ethylene flow rate (kg/hr) + Ethanol flow rate in terms of ethylene (kg/hr) + Flow rate (kg/hr) of olefin having from 4 to 6 carbon atoms + Flow rate (kg/hr) of oxygenate having from 1 to 6 carbon atoms Ethanol flow rate in terms of ethylene (kg/hr) = Ethanol flow rate (kg/hr) $\times$ Molecular weight (g/mol) of ethylene / Molecular weight (g/mol) of ethanol

(Propylene yield)

The propylene yield indicates the selectivity of reaction step for propylene and is calculated according to the following expression.

Propylene yield (% by mass) = Mass per hour of propylene produced in reaction step (kg/hr) / Mass flow rate of effective raw material fed (kg/hr) $\times$ 100

(Aromatic yield)

The aromatic yield indicates selectivity for an aromatic compound in a reaction step and was calculated according to the following expression.

Aromatic yield (% by mass) = Mass per hour of aromatic compound produced in reaction step (kg/hr)/ Mass flow rate of effective raw material fed (kg/hr) $\times$ 100

Mass flow rate of effective raw material fed (kg/hr) = Ethylene flow rate (kg/hr) + Ethanol flow rate in terms of ethylene (kg/hr) + Flow rate (kg/hr) of olefin having from 4 to 6 carbon atoms + Flow rate (kg/hr) of oxygenate having from 1 to 6 carbon atoms Ethanol flow rate in terms of ethylene (kg/hr) = Ethanol flow rate (kg/hr) × Molecular weight (g/mol) of ethylene / Molecular weight (g/mol) of ethanol

(Yield maintenance rate)

In Examples 1 to 6 and Comparative Example 1, reaction for 48 hours was regarded as one cycle, and the maintenance rate of the ethylene conversion rate after 24 hours, the maintenance rate of the ethylene conversion rate after 48 hours, the maintenance rate of the propylene yield after 24 hours, the maintenance rate of the propylene yield after 48 hours, the maintenance rate of the aromatic yield after 24 hours, and the maintenance rate of the aromatic yield after 48 hours were calculated according to the expressions give below.

In Examples 7 to 14 and Comparative Examples 3 to 5, a catalyst after the completion of the first cycle was treated by regeneration to remove accumulating coke, and the second cycle was carried out under similar conditions. The maintenance rate of the ethylene conversion rate after regeneration, the maintenance rate of the propylene yield after regeneration, and the maintenance rate of the aromatic yield after regeneration were calculated according to the expressions given below.

Maintenance rate (% by mass) of ethylene conversion rate after 24 hours = Ethylene conversion rate (% by mass) after lapse of 24 hours from start of reaction of first cycle / Ethylene conversion rate (% by mass) after lapse of 3 hours from start of reaction of first cycle × 100 Maintenance rate (% by mass) of ethylene conversion rate after 48 hours = Ethylene conversion rate (% by mass) after lapse of 48 hours from start of reaction of first cycle / Ethylene conversion rate (% by mass) after lapse of 3 hours from start of reaction of first cycle × 100 Maintenance rate (% by mass) of ethylene conversion rate after regeneration = Ethylene conversion rate (% by mass) after lapse of 3 hours from start of reaction of second cycle / Ethylene conversion rate (% by mass) after lapse of 3 hours from start of reaction of first cycle × 100 Maintenance rate (% by mass) of propylene yield after 24 hours = Propylene yield (% by mass) after lapse of 24 hours from start of reaction of first cycle / Propylene yield (% by mass) after lapse of 3 hours from start of reaction of first cycle × 100 Maintenance rate (% by mass) of propylene yield after 48 hours = Propylene yield (% by mass) after lapse of 48 hours from start of reaction of first cycle / Propylene yield (% by mass) after lapse of 3 hours from start of reaction of first cycle × 100 Maintenance rate (% by mass) of propylene yield after regeneration = Propylene yield (% by mass) after lapse of 3 hours from start of reaction of second cycle / Propylene yield (% by mass) after lapse of 3 hours from start of reaction of first cycle × 100 Maintenance rate (% by mass) of aromatic yield after 24 hours = Aromatic yield (% by mass) after lapse of 24 hours from start of reaction of first cycle / Aromatic yield (% by mass) after lapse of 3 hours from start of reaction of first cycle × 100 Maintenance rate (% by mass) of aromatic yield after 48 hours = Aromatic yield (% by mass) after lapse of 48 hours from start of reaction of first cycle / Aromatic yield (% by mass) after lapse of 3 hours from start of reaction of first cycle × 100 Maintenance rate (% by mass) of aromatic yield after regeneration = Aromatic yield (% by mass) after lapse of 3 hours from start of reaction of second cycle / Aromatic yield (% by mass) after lapse of 3 hours from start of reaction of first cycle × 100

[Production Example 1: Preparation of zeolite-containing catalyst 1]

Clay obtained from 70 parts by mass of medium pore-size zeolite proton-type ZSM-5 (molar ratio of silica/alumina: 980) and 89 parts by mass of a colloidal silica solution (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 2.1 mm and a length of from 4 to 6 mm. The obtained extrudate was calcined at 600°C for 5 hours to obtain zeolite-containing catalyst 1. In this respect, the content of a sodium element contained in the zeolite-containing catalyst was 55 ppm by mass.

[Production Example 2: Preparation of zeolite-containing catalyst 2]

Clay obtained from 70 parts by mass of medium pore-size zeolite proton-type ZSM-5 (molar ratio of silica/alumina: 980) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 2.1 mm and a length of from 4 to 6 mm. The obtained extrudate was calcined at 600°C for 5 hours to obtain a catalyst precursor. The obtained catalyst precursor was stirred for 1 hour in a 0.1 N aqueous sodium nitrate solution, then filtered, washed, and calcined at 600°C for 5 hours to obtain a sodium-replaced product. The sodium-replaced product was stirred for 1 hour in a 0.01 N aqueous silver nitrate solution, subjected to filtration and washing three repeated times, and calcined at 600°C for 5 hours to obtain a silver-replaced product. A water vapor-air mixed gas containing 80% by volume of water vapor was fed and circulated into the silver-replaced product under conditions involving a pressure of 0.1 MPa and a temperature of 600°C for 24 hours to obtain zeolite-containing catalyst 2. In this respect, the content of a silver element contained in the zeolite-containing catalyst was 0.16% by mass, and the content of a sodium element was 46 ppm by mass.

[Production Example 3: Method for preparing zeolite-containing catalyst 3]

Clay obtained from 70 parts by mass of medium pore-size zeolite proton-type ZSM-5 (molar ratio of silica/alumina: 980) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 2.1 mm and a length of from 4 to 6 mm. The obtained extrudate was allowed to support a predetermined amount of an aqueous diammonium hydrogen phosphate solution to obtain a phosphorus-supported product. The obtained phosphorus-supported product was calcined at 600°C for 5 hours in an air atmosphere. The calcined product was filled into a reactor, and a water vapor-nitrogen mixed gas containing 80% by volume of water vapor was fed and circulated thereinto under conditions involving a pressure of 0.1 MPa and a temperature of 600°C for 24 hours to obtain zeolite-containing catalyst 3. In this respect, the content of a phosphorus element contained in the zeolite-containing catalyst was 0.032% by mass, and the content of a sodium element was 76 ppm by mass.

[Production Example 4: Preparation of zeolite-containing catalyst 4]

Clay obtained from 70 parts by mass of medium pore-size zeolite proton-type ZSM-5 (molar ratio of silica/alumina: 212) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 1.6 mm and a length of from 4 to 6 mm. The obtained extrudate was allowed to support a predetermined amount of an aqueous diammonium hydrogen phosphate solution to obtain a phosphorus-supported product. The obtained phosphorus-supported product was calcined at 600°C for 5 hours in an air atmosphere. The calcined product was filled into a reactor, and a water vapor-nitrogen mixed gas containing 80% by volume of water vapor was fed and circulated thereinto under conditions involving a pressure of 0.1 MPa and a temperature of 600°C for 24 hours to obtain zeolite-containing catalyst 4. In this respect, the content of a phosphorus element contained in the zeolite-containing catalyst was 0.14% by mass, and the content of a sodium element was 253 ppm by mass.

[Production Example 5: Preparation of zeolite-containing catalyst 5]

Clay obtained from 70 parts by mass of medium pore-size zeolite proton-type ZSM-5 (molar ratio of silica/alumina: 302) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 1.6 mm and a length of from 4 to 6 mm. The obtained extrudate was allowed to support a predetermined amount of an aqueous diammonium hydrogen phosphate solution to obtain a phosphorus-supported product. The obtained phosphorus-supported product was calcined at 600°C for 5 hours in an air atmosphere. The calcined product was filled into a reactor, and a water vapor-nitrogen mixed gas containing 80% by volume of water vapor was fed and circulated thereinto under conditions involving a pressure of 0.1 MPa and a temperature of 600°C for 24 hours to obtain zeolite-containing catalyst 5. In this respect, the content of a phosphorus element contained in the zeolite-containing catalyst was 0.085% by mass, and the content of a sodium element was 98 ppm by mass.

[Production Example 6: Preparation of zeolite-containing catalyst 6]

Clay obtained from 70 parts by mass of medium pore-size zeolite proton-type ZSM-5 (molar ratio of silica/alumina: 150) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 1.6 mm and a length of from 4 to 6 mm. The obtained extrudate was dried at 350°C for 5 hours and the calcined at 600°C for 5 hours to obtain zeolite-containing catalyst 6. In this respect, the content of a sodium element contained in the zeolite-containing catalyst was 141 ppm by mass.

[Production Example 7: Preparation of zeolite-containing catalyst 7]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours to obtain a catalyst precursor. The obtained catalyst precursor was stirred for 1 hour in a 0.01 N aqueous silver nitrate solution, subjected to filtration and washing three repeated times, and calcined at 600°C for 5 hours to obtain zeolite-containing catalyst 7. In this respect, the content of a silver element contained in the zeolite-containing catalyst was 0.2339% by mass, and the content of a sodium element was 10 ppm by mass.

[Production Example 8: Preparation of zeolite-containing catalyst 8]

A water vapor-air mixed gas containing 80% by volume of water vapor was fed and circulated into the zeolite-containing catalyst 7 under conditions involving a pressure of 0.1 MP and a temperature of 600°C for 24 hours to obtain zeolite-containing catalyst 8.

[Production Example 9: Preparation of zeolite-containing catalyst 9]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours to obtain a catalyst precursor. The obtained extrudate was stirred for 1 hour in a 0.002 N aqueous silver nitrate solution, subjected to filtration and washing three repeated times, and calcined at 600°C for 5 hours to obtain zeolite-containing catalyst 9. In this respect, the content of a silver element contained in the zeolite-containing catalyst was 0.1360% by mass, and the content of a sodium element was 14 ppm by mass.

[Production Example 10: Preparation of zeolite-containing catalyst 10]

A water vapor-air mixed gas containing 80% by volume of water vapor was fed and circulated into the zeolite-containing catalyst 9 under conditions involving a pressure of 0.1 MPa and a temperature of 600°C for 24 hours to obtain zeolite-containing catalyst 10.

[Production Example 11: Preparation of zeolite-containing catalyst 11]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours to obtain a catalyst precursor. The obtained catalyst precursor was stirred for 1 hour in a 0.1 N aqueous sodium nitrate solution, then filtered, washed, and calcined at 600°C for 5 hours to obtain a sodium-replaced product. The sodium-replaced product was stirred for 1 hour in a 0.01 N aqueous silver nitrate solution, subjected to filtration and washing three repeated times, and calcined at 600°C for 5 hours to obtain a silver-replaced product. A water vapor-air mixed gas containing 80% by volume of water vapor was fed and circulated into the silver-replaced product under conditions involving a pressure of 0.1 MPa and a temperature of 600°C for 24 hours to obtain zeolite-containing catalyst 11. In this respect, the content of a silver element contained in the zeolite-containing catalyst was 0.2641% by mass, and the content of a sodium element was 38 ppm by mass.

[Production Example 12: Preparation of zeolite-containing catalyst 12]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours, stirred for 1 hour in a 0.001 N aqueous silver nitrate solution, subjected to filtration and washing three repeated times, and further calcined at 600°C for 5 hours to obtain zeolite-containing catalyst 12. In this respect, the content of a silver element contained in the zeolite-containing catalyst was 0.1899% by mass, and the content of a sodium element was 61 ppm by mass.

[Production Example 13: Preparation of zeolite-containing catalyst 13]

Clay obtained from 70 parts by mass of medium pore-size zeolite proton-type ZSM-5 (molar ratio of silica/alumina: 980) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 2.1 mm and a length of from 4 to 6 mm. The obtained extrudate was calcined at 600°C for 5 hours, dipped in 100 parts by mass of 0.1 N nitric acid based on 10 parts by mass of the extrudate at room temperature for 1 hour, and then washed with water to obtain zeolite-containing catalyst 13. In this respect, the content of a sodium element contained in the zeolite-containing catalyst was 18 ppm by mass.

[Production Example 14: Preparation of zeolite-containing catalyst 14]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours, dipped in 100 parts by mass of 0.1 N nitric acid based on 10 parts by mass of the extrudate at room temperature for 1 hour, and then washed with water to obtain a catalyst precursor. The obtained precursor was filled into a reactor, and a water vapor-nitrogen mixed gas containing 80% by volume of water vapor was fed and circulated thereinto under conditions involving a pressure of 0.1 MPa and a temperature of 600°C for 48 hours to obtain zeolite-containing catalyst 14. In this respect, the content of a sodium element contained in the zeolite-containing catalyst was 21 ppm by mass.

[Production Example 15: Preparation of zeolite-containing catalyst 15]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours, dipped in 100 parts by mass of 0.1 N nitric acid based on 10 parts by mass of the extrudate at room temperature for 1 hour, and then washed with water to obtain a catalyst precursor. The obtained precursor was allowed to support a predetermined amount of an aqueous diammonium hydrogen phosphate solution to obtain a phosphorus-supported product. The obtained phosphorus-supported product was calcined at 600°C for 5 hours in an air atmosphere to obtain zeolite-containing catalyst 15. In this respect, the content of a phosphorus element contained in the zeolite-containing catalyst was 0.1687% by mass, and the content of a sodium element was 18 ppm by mass.

[Production Example 16: Preparation of zeolite-containing catalyst 16]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours, dipped in 100 parts by mass of 0.1 N nitric acid based on 10 parts by mass of the extrudate at room temperature for 1 hour, and then washed with water to obtain a catalyst precursor. The obtained precursor was allowed to support a predetermined amount of an aqueous diammonium hydrogen phosphate solution to obtain a phosphorus-supported product. The obtained phosphorus-supported product was calcined at 600°C for 5 hours in an air atmosphere to obtain zeolite-containing catalyst 16. In this respect, the content of a phosphorus element contained in the zeolite-containing catalyst was 0.0371% by mass, and the content of a sodium element was 7 ppm by mass.

[Production Example 17: Preparation of zeolite-containing catalyst 17]

Clay obtained from 80 parts by mass of medium pore-size zeolite proton-type ZSM-5 (molar ratio of silica/alumina: 850) and 59 parts by mass of colloidal silica (33.8% by mass of silica, sodium content based on the total amount of the colloidal silica solution: 28 ppm by mass) corresponding to 20 parts by mass of silica was kneaded, followed by extrusion molding to obtain an extrudate adjusted to a diameter of 2.6 mm and a length of from 4 to 6 mm. The obtained extrudate was calcined at 600°C for 5 hours to obtain a catalyst precursor. The obtained catalyst precursor was stirred for 1 hour in a 0.1 N aqueous sodium nitrate solution, then filtered, washed, and calcined at 600°C for 5 hours to obtain a sodium-replaced product. The sodium-replaced product was stirred for 1 hour in a 0.002 N aqueous silver nitrate solution, subjected to filtration and washing, and calcined at 600°C for 5 hours to obtain zeolite-containing catalyst 17. In this respect, the content of a silver element contained in the zeolite-containing catalyst was 0.1291 % by mass, and the content of a sodium element was 339 ppm by mass.

[Production Example 18: Preparation of zeolite-containing catalyst 18]

Clay obtained from 70 parts by mass of medium pore-size zeolite proton-type ZSM-5 (molar ratio of silica/alumina: 980) and 89 parts by mass of colloidal silica (33.8% by mass of silica, sodium content based on the total amount of the colloidal silica solution: 28 ppm by mass) corresponding to 30 parts by mass of silica was kneaded, followed by extrusion molding to obtain an extrudate adjusted to a diameter of 2.1 mm and a length of from 4 to 6 mm. The obtained extrudate was calcined at 600°C for 5 hours to obtain zeolite-containing catalyst 18. In this respect, the content of a sodium element contained in the zeolite-containing catalyst was 156 ppm by mass.

[Example 1]

(Evaluation of physical properties of catalyst)

The zeolite-containing catalyst 1 had an initial TPD acid content of 10.7 μmol/g and an acid content maintenance rate of 78.1%. Each physical property of the catalyst obtained by characterization are shown in Table 1.

(Reaction step)

A mixed raw material containing 19.2% by mass of ethylene, 48.1% by mass of ethanol, 18.1% by mass of water, and 14.6% by mass of 1-butene was fed at WHSV of 2.2, a pressure of 0.15 MPaG, and a catalyst bed inlet temperature of 530°C into a reactor filled with the zeolite-containing catalyst 1, and the reaction step was carried out for 48 hours. The propylene yield and the aromatic yield after a lapse of 3 hours from the start of reaction were 18.4% by mass and 3.5% by mass, respectively. The maintenance rate of the propylene yield and the maintenance rate of the aromatic yield after a lapse of 48 hours were 91.7% and 60.2%, respectively. The reaction results are shown in Table 1 and Figure 4.

[Example 2]

(Regeneration step)

While the zeolite-containing catalyst 1 used in the reaction step in Example 1 was filled in the reactor, the circulated gas was switched from the raw material to nitrogen diluted gas, and the regeneration step was carried out under conditions of procedures 1 to 5 given below. The regenerated zeolite-containing catalyst 1 (regenerated catalyst) was used again in the reaction step.

Procedure 1) Temperature: 480°C, oxygen concentration: 1%, 1 hr

Procedure 2) Temperature: 520°C, oxygen concentration: 1%, 3 hr

Procedure 3) Temperature: 550°C, oxygen concentration: 1%, 3 hr

Procedure 4) Temperature: 550°C, oxygen concentration: 5%, 1 hr

Procedure 5) Temperature: 580°C, oxygen concentration: 5%, 2 hr

(Reaction step: second cycle)

The reaction step carried out in the same manner as in Example 1 except that the regenerated catalyst was used. The maintenance rate of the propylene yield and the maintenance rate of the aromatic yield after a lapse of 48 hours were 91.8% and 63.1%, respectively. The reaction results are shown in Figure 4.

(Reaction step: third cycle)

After the reaction step of the second cycle was carried out, the regeneration step was carried out in the same manner as above, and the reaction step of the third cycle was carried out. The maintenance rate of the propylene yield and the maintenance rate of the aromatic yield after a lapse of 48 hours were 92.4% and 57.1%, respectively. The reaction results are shown in Figure 4.

[Example 3]

This Example was carried out in the same manner as in Example 1 except that the zeolite-containing catalyst 2 was used. The zeolite-containing catalyst 2 had an initial TPD acid content of 10.3 $\mu$mol/g and an acid content maintenance rate of 93.5%. The maintenance rate of the propylene yield and the maintenance rate of the aromatic yield after a lapse of 48 hours were 93.6% and 61.4%, respectively. Each physical property of the catalyst obtained by characterization and the reaction results are shown in Table 1.

[Example 4]

This Example was carried out in the same manner as in Example 1 except that the zeolite-containing catalyst 3 was used. The zeolite-containing catalyst 3 had an initial TPD acid content of 11.3 $\mu$mol/g and an acid content maintenance rate of 81.5%. The maintenance rate of the propylene yield and the maintenance rate of the aromatic yield after a lapse of 48 hours were 92.1% and 61.2%, respectively. Each physical property of the catalyst obtained by characterization and the reaction results are shown in Table 1.

[Example 5]

This Example was carried out in the same manner as in Example 1 except that the zeolite-containing catalyst 4 was used. The zeolite-containing catalyst 4 had an initial TPD acid content of 40.9 $\mu$mol/g and an acid content maintenance rate of 73.1%. The maintenance rate of the propylene yield and the maintenance rate of the aromatic yield after a lapse of 48 hours were 79.4% and 50.1%, respectively. Each physical property of the catalyst obtained by characterization and the reaction results are shown in Table 1.

[Example 6]

This Example was carried out in the same manner as in Example 1 except that the zeolite-containing catalyst 5 was used. The zeolite-containing catalyst 5 had an initial TPD acid content of 32.1 $\mu$mol/g and an acid content maintenance rate of 73.1%. The maintenance rate of the propylene yield and the maintenance rate of the aromatic yield after a lapse of 48 hours were 78.1% and 52.1%, respectively. Each physical property of the catalyst obtained by characterization and the reaction results are shown in Table 1.

[Comparative Example 1]

This Comparative Example was carried out in the same manner as in Example 1 except that the zeolite-containing catalyst 6 was used. The zeolite-containing catalyst 6 had an initial TPD acid content of 86.2 μmol/g and an acid content maintenance rate of 44.8%. The propylene yield and the aromatic yield after a lapse of 3 hours from the start of reaction were 18.2% by mass and 3.1% by mass, respectively. The maintenance rate of the propylene yield and the maintenance rate of the aromatic yield after a lapse of 48 hours were 54.7% and 42.9%, respectively. Each physical property of the catalyst obtained by characterization and the reaction results are shown in Table 1.

The comparison of Examples with Comparative Example demonstrated that a zeolite-containing catalyst that exhibits an initial TPD acid content of more than a given level markedly decreases an acid content maintenance rate. This comparison also demonstrated that a zeolite-containing catalyst having a low acid content maintenance rate decreases an average yield of a target compound and cannot maintain the yield.

[Comparative Example 2]

The zeolite-containing catalyst 6 used in Comparative Example 1 was subjected to the regeneration step in the same manner as in Example 2. The reaction step was performed again using the obtained regenerated catalyst. The propylene yield and the aromatic yield after a lapse of 3 hours from the start of reaction were 12.2% by mass and 1.1% by mass, respectively, which were markedly reduced as compared with Comparative Example 1.

[Table 1]

Table 1

| Example | | 1 | 3 | 4 | 5 | 6 | Comparative Example 1 |
|---|---|---|---|---|---|---|---|
| Zeolite-containing catalyst | | 1 | 2 | 3 | 4 | 5 | 6 |
| Zeolite species | | MFI | MFI | MFI | MFI | MFI | MFI |
| Molar ratio of silica/alumina | [-] | 980 | 980 | 980 | 212 | 302 | 150 |
| Binder | | Silica | Silica | Silica | Silica | Silica | Silica |
| Binder content | [% by mass] | 30 | 30 | 30 | 30 | 30 | 30 |
| Supported element | | - | Ag | P | P | P | - |
| Content of supported element | [% by mass] | - | 0.16 | 0.032 | 0.14 | 0.085 | - |
| Mesopore volume | [cc/g] | 0.32 | 0.32 | 0.32 | 0.23 | 0.28 | 0.38 |
| Content of Si | [% by mass] | 46.0 | 46.0 | 46.0 | 43.4 | 42.3 | 45.5 |
| Content of Al | [% by mass] | 0.07 | 0.07 | 0.07 | 0.28 | 0.20 | 0.43 |
| Mass ratio of Si/Al | [-] | 657 | 657 | 657 | 155 | 212 | 106 |
| Content of Na | [ppm by mass] | 55 | 46 | 76 | 253 | 98 | 141 |
| Molar ratio of Na/Al | [-] | 0.09 | 0.08 | 0.13 | 0.11 | 0.06 | 0.04 |
| Initial TPD acid content per catalyst weight | [μmol/g] | 10.7 | 10.3 | 11.3 | 40.9 | 32.1 | 86.2 |
| Acid content after 1.0-hr STM treatment | [μmol/g] | 10.6 | 11.6 | 9.8 | 22.7 | 22.1 | 38.9 |
| Acid content after 6.0-hr STM treatment | [μmol/g] | 8.2 | 10.9 | 7.9 | 16.6 | 16.1 | 17.4 |
| Acid content maintenance rate | [%] | 78.1% | 93.5% | 81.5% | 73.1% | 73.1% | 44.8% |
| Initial TPD acid content per zeolite weight | [μmol/g] | 15.3 | 14.7 | 16.1 | 58.5 | 45.9 | 123.1 |

(continued)

| Example | | 1 | 3 | 4 | 5 | 6 | Comparative Example 1 |
|---|---|---|---|---|---|---|---|
| Maintenance rate of ethylene conversion rate after 24 hours | [%] | 95.7 | 96.8 | 92.1 | 90.8 | 91.1 | 84.6 |
| Maintenance rate of ethylene conversion rate after 48 hours | [%] | 93.0 | 95.4 | 89.9 | 86.3 | 85.0 | 77.6 |
| Maintenance rate of propylene yield after 24 hours | [%] | 94.2 | 95.9 | 94.5 | 86.2 | 100.5 | 67.4 |
| Maintenance rate of propylene yield after 48 hours | [%] | 91.7 | 93.6 | 92.1 | 79.4 | 78.1 | 54.7 |
| Maintenance rate of aromatic yield after 24 hours | [%] | 83.2 | 71.2 | 80.3 | 79.7 | 70.8 | 49.0 |
| Maintenance rate of aromatic yield after 48 hours | [%] | 60.2 | 61.4 | 61.2 | 50.1 | 52.1 | 42.9 |
| Amount of coke | [ppm by mass] | 146 | 162 | 133 | 289 | 255 | 323 |

[Example 7]

(Evaluation of physical properties of catalyst)

Each physical property of the zeolite-containing catalyst 7 obtained by characterization are shown in Table 2.

(Reaction step)

A mixed raw material containing 28.1% by mass of ethylene, 57.1% by mass of ethanol, and 14.8% by mass of water was fed at WHSV of 2.2, a pressure of 0.15 MPaG, and a catalyst bed inlet temperature of 500°C into a reactor filled with the zeolite-containing catalyst 7, and the reaction step was carried out for 48 hours. The ethylene yield, the propylene yield, and the aromatic yield after a lapse of 3 hours from the start of reaction were 22.6% by mass (ethylene conversion rate: 77.4% by mass), 21.5% by mass, and 7.9% by mass, respectively. The ethylene yield, the propylene yield, and the aromatic yield after a lapse of 24 hours were 26.5% by mass (ethylene conversion rate: 73.5% by mass), 21.3% by mass, and 6.3% by mass, respectively.

Subsequently, the zeolite-containing catalyst 7 used in the evaluation of the reaction for 48 hours was discharged from the reactor and calcined at 580°C for 3 hours under air circulation to regenerate the catalyst. Reaction evaluation was carried out under the same conditions as in the reaction evaluation described above using the obtained catalyst thus regenerated. The ethylene yield, the propylene yield, and the aromatic yield after a lapse of 3 hours from the start of reaction were 30.2% by mass (ethylene conversion rate: 69.8% by mass), 21.3% by mass, and 5.2% by mass, respectively. The maintenance rate of each yield is shown in Table 2.

[Examples 8 to 16 and Comparative Examples 3 to 5]

Evaluation of physical properties of each catalyst and reaction evaluation were carried out in the same manner as in Example 7 except that the catalyst described in Table 2 was used. The results are shown in Table 2.

The comparison of Comparative Example 3 with Examples demonstrated that when zeolite has a large TPD acid content, compositional variation of a product is large because an ethylene conversion rate after a lapse of 24 hours from the start of reaction and a propylene yield after a lapse of 24 hours from the start of reaction cannot be maintained.

The comparison of Comparative Examples 4 and 5 with Examples demonstrated that when a zeolite-containing catalyst has a large content of a sodium element or a large molar ratio of Na/Al, an ethylene conversion rate after a lapse of 24 hours from the start of reaction, a propylene yield after a lapse of 24 hours from the start of reaction, an ethylene conversion rate after regeneration, and a propylene yield after regeneration cannot be maintained and the amount of coke generated is large.

[Table 2]

EP 4 660 178 A1

Table 2

| Example | | 7 | 8 | 9 | 10 | 11 | 12 | 13 | 14 | 15 | 16 | Comparative Example 3 | Comparative Example 4 | Comparative Example 5 |
|---|---|---|---|---|---|---|---|---|---|---|---|---|---|---|
| Zeolite-containing catalyst | | 7 | 8 | 9 | 10 | 11 | 12 | 13 | 14 | 15 | 16 | 6 | 17 | 18 |
| Zeolite species | | MFI | MFI | MFI | MFI | : MFI | MFI | MFI | MFI | MFI | MFI | MFI | MFI | MFI |
| Molar ratio of silica/alumina | [-] | 850 | 850 | 850 | 850 | 850 | 850 | 980 | 850 | 850 | 850 | 150 | 850 | 980 |
| Binder | | Silica | Silica | Silica | Silica | Silica | Silica | Silica | Silica | Silica | Silica | Silica | Silica | Silica |
| Binder content | [% by mass] | 20 | 20 | 20 | 20 | 20 | 20 | 30 | 20 | 20 | 20 | 30 | 20 | 30 |
| Supported element | | Ag | Ag | Ag | Ag | Ag | Ag | - | - | P | P | - | Ag | - |
| Content of supported element | [% by mass] | 0.2339 | 0.2339 | 0.1360 | 0.1360 | 0.2641 | 0.1899 | - | - | 0.1687 | 0.0371 | - | 0.1291 | - |
| Content of Si | [% by mass] | 46.55 | 46.55 | 46.60 | 46.60 | 46.54 | 46.57 | 46.69 | 46.67 | 46.31 | 46.59 | 45.50 | 46.59 | 46.68 |
| Content of Al | [% by mass] | 0.08 | 0.08 | 0.08 | 0.08 | 0.08 | 0.08 | 0.06 | 0.08 | 0.08 | 0.08 | 0.43 | 0.08 | 0.06 |
| Mass ratio of Si/Al | [-] | 582 | 582 | 583 | 583 | 582 | 582 | 778 | 583 | 579 | 582 | 106 | 582 | 778 |
| Content of Na | [ppm by mass] | 10 | 10 | 14 | 14 | 38 | 61 | 18 | 21 | 18 | 7 | 141 | 339 | 156 |
| Molar ratio of Na/Al | [-] | 0.015 | 0.015 | 0.021 | 0.021 | 0.056 | 0.089 | 0.035 | 0.031 | 0.026 | 0.010 | 0.038 | 0.497 | 0.305 |
| TPD acid content per catalyst weight | [$\mu$mol/g] | 23.3 | 18.6 | 19.7 | 13.4 | 20.5 | 15.2 | 6.1 | 2.6 | 23.4 | 21.5 | 86.2 | 12.8 | 9.2 |
| TPD acid content per zeolite weight | [$\mu$mol/g] | 29.1 | 23.3 | 24.6 | 16.7 | 25.6 | 19.0 | 8.7 | 3.3 | 29.3 | 26.9 | 86.2 | 16.0 | 13.1 |
| Strength | [N/mm] | 3.6 | 9.0 | 4.9 | 8.7 | 8.9 | 8.8 | 4.7 | 4.9 | 5.5 | 6.8 | 3.5 | 3.7 | 3.7 |

| Example | | 7 | 8 | 9 | 10 | 11 | 12 | 13 | 14 | 15 | 16 | Comparative Example 3 | Comparative Example 4 | Comparative Example 5 |
|---|---|---|---|---|---|---|---|---|---|---|---|---|---|---|
| Maintenance rate of ethylene conversion rate after 24 hours | [%] | 95.0 | 95.0 | 103.2 | 93.9 | 97.4 | 89.8 | 105.4 | 98.7 | 89.5 | 98.8 | 71.0 | 45.4 | 73.7 |
| Maintenance rate of ethylene conversion rate after regeneration | [%] | 90.2 | 89.8 | 103.5 | 86.3 | 89.1 | 88.2 | 83.3 | 96.1 | 94.3 | 96.1 | 67.0 | 68.0 | 92.9 |
| Maintenance rate of propylene yield after 24 hours | [%] | 99.1 | 97.7 | 102.2 | 95.0 | 99.2 | 92.5 | 91.1 | 97.9 | 97.4 | 100.6 | 79.0 | 51.2 | 81.0 |
| Maintenance rate of propylene yield after regeneration | [%] | 99.1 | 94.1 | 103.4 | 93.2 | 98.5 | 90.5 | 84.9 | 98.5 | 100.2 | 97.9 | 99.0 | 71.2 | 97.3 |
| Maintenance rate of aromatic yield after 24 hours | [%] | 79.7 | 81.6 | 94.7 | 79.0 | 90.6 | 66.1 | 69.7 | 92.7 | 62.7 | 93.6 | 67.2 | 19.0 | 50.3 |
| Maintenance rate of aromatic yield after regeneration | [%] | 65.8 | 69.5 | 86.4 | 66.6 | 87.3 | 59.0 | 83.6 | 90.1 | 80.8 | 88.7 | 80.1 | 36.7 | 83.5 |
| Amount of coke | [ppm by mass] | 166 | 133 | 117 | 100 | 137 | 198 | 49 | 41 | 111 | 104 | 291 | 512 | 272 |

EP 4 660 178 A1

31

**Reference Sings List**

1: reactor, 2: first distillation column, 3: cooler, 4: oil-water separator

**Claims**

1. A method for converting ethanol, comprising

   a reaction step of feeding a mixed raw material containing ethanol into a reactor having a fixed bed filled with a zeolite-containing catalyst to obtain a reaction gas containing an olefin having 3 or more carbon atoms and water, wherein
   a zeolite contained in the zeolite-containing catalyst has an oxygen 10-membered ring structure,
   a molar ratio of Na/Al of the zeolite-containing catalyst is from 0.0050 to 0.25, and
   an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

2. The method for converting ethanol according to claim 1, wherein the acid content Ac (unit: $\mu$mol/g) per weight of the zeolite-containing catalyst satisfies the following expression (1):

$$Ac \leq 25 - 60 \times [\text{Molar ratio of Na/Al}] \ ... \ (1).$$

3. The method for converting ethanol according to claim 1, wherein the acid content per weight of the zeolite-containing catalyst is 0.5 $\mu$mol/g or more.

4. The method for converting ethanol according to claim 1, wherein an acid content per weight of the zeolite determined from an amount of ammonia desorbed at from 100 to 650°C in ammonia temperature-programmed desorption measurement of the zeolite-containing catalyst is 75 $\mu$mol/g or less.

5. The method for converting ethanol according to claim 1, wherein a content of sodium contained in the zeolite-containing catalyst is 100 ppm by mass or less.

6. The method for converting ethanol according to claim 1, wherein a Si/Al mass ratio of the zeolite-containing catalyst is from 300 to 3000.

7. The method for converting ethanol according to claim 1, wherein a molar ratio of silica/alumina of the zeolite in the zeolite-containing catalyst is from 600 to 2000.

8. The method for converting ethanol according to claim 1, wherein the zeolite-containing catalyst comprises at least one dope element selected from the group consisting of phosphorus and an element of group 11.

9. The method for converting ethanol according to claim 8, wherein a content of the dope element is 2.0% by mass or less based on the total amount of the zeolite-containing catalyst.

10. The method for converting ethanol according to claim 1, wherein an aluminum content is from 0.01% by mass to 1% by mass based on the total amount of the zeolite-containing catalyst.

11. The method for converting ethanol according to claim 1, wherein the mixed raw material contains ethylene.

12. The method for converting ethanol according to claim 1, wherein an ethanol content of the mixed raw material is 30% by mass or more based on the mixed raw material.

13. The method for converting ethanol according to claim 1, wherein a molar ratio of ethylene/ethanol of the mixed raw material is from 0.20 to 2.50.

14. The method for converting ethanol according to claim 1, further comprising a regeneration step of contacting the zeolite-containing catalyst subjected to the reaction step with an oxygen-containing gas heated to 400°C or higher, and subjecting the zeolite-containing catalyst again to the reaction step.

15. A method for producing propylene, comprising
a propylene-separating step of separating a fraction mainly comprising propylene from a reaction gas obtained by the method for converting ethanol according to any one of claims 1 to 14.

16. A method for producing an aromatic compound, comprising
an aromatic compound-separating step of separating a fraction mainly comprising an aromatic compound from a reaction gas obtained by the method for converting ethanol according to any one of claims 1 to 14.

17. A zeolite-containing catalyst, wherein

a zeolite contained in the zeolite-containing catalyst has an oxygen 10-membered ring structure,
a molar ratio of Na/Al of the zeolite-containing catalyst is from 0.0050 to 0.25, and
an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

18. The zeolite-containing catalyst according to claim 17, wherein a mass ratio of Si/Al of the zeolite-containing catalyst is from 300 to 3000.

19. The zeolite-containing catalyst according to claim 17, wherein the zeolite-containing catalyst is a catalyst for obtaining an olefin having 3 or more carbon atoms from a mixed raw material containing ethanol.

20. The zeolite-containing catalyst according to claim 17, wherein the zeolite-containing catalyst comprises at least one dope element selected from the group consisting of phosphorus and an element of group 11.

21. The zeolite-containing catalyst according to claim 20, wherein a content of the dope element is 2.0% by mass or less based on the total amount of the zeolite-containing catalyst.

22. The zeolite-containing catalyst according to claim 17, wherein an aluminum content is from 0.01% by mass to 1% by mass based on the total amount of the zeolite-containing catalyst.

23. A method for producing a zeolite-containing catalyst, comprising a steaming step of contacting the zeolite-containing catalyst with water vapor at a steaming temperature of 450°C or higher, wherein

a zeolite contained in the zeolite-containing catalyst is zeolite having an oxygen 10-membered ring structure,
a molar ratio of Na/Al of the zeolite-containing catalyst is from 0.0050 to 0.25, and
an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

24. A method for producing a hydrocarbon, comprising

a reaction step of feeding a raw material containing ethanol into a reactor having a fixed bed filled with a zeolite-containing catalyst to obtain a reaction gas containing an olefin having 3 or more carbon atoms and water, wherein
a zeolite contained in the zeolite-containing catalyst is zeolite having an oxygen 10-membered ring structure,
a molar ratio of Na/Al of the zeolite-containing catalyst is from 0.0050 to 0.25, and
an acid content per weight of the zeolite-containing catalyst determined from an amount of ammonia desorbed at from 100 to 650°C in ammonia temperature-programmed desorption measurement of the zeolite-containing catalyst is 75 $\mu$mol/g or less.

FIG.1

FIG.2

FIG.3

FIG.4

## INTERNATIONAL SEARCH REPORT

| | International application No. |
|---|---|
| | **PCT/JP2024/002643** |

**A. CLASSIFICATION OF SUBJECT MATTER**

*C07C 1/24*(2006.01)i; *B01J 29/44*(2006.01)i; *B01J 37/10*(2006.01)i; *B01J 38/12*(2006.01)i; *C07B 61/00*(2006.01)i; *C07C 2/12*(2006.01)i; *C07C 7/00*(2006.01)i; *C07C 11/04*(2006.01)i; *C07C 11/06*(2006.01)i; *C07C 15/02*(2006.01)i; *C07C 15/46*(2006.01)i

FI:  C07C1/24; C07C11/06; C07C15/02; B01J38/12 B; B01J29/44 M; B01J37/10; C07C15/46; C07C7/00; C07C11/04; C07B61/00 300; C07C2/12

According to International Patent Classification (IPC) or to both national classification and IPC

**B. FIELDS SEARCHED**

Minimum documentation searched (classification system followed by classification symbols)

C07C1/24; B01J29/44; B01J37/10; B01J38/12; C07B61/00; C07C2/12; C07C7/00; C07C11/04; C07C11/06; C07C15/02; C07C15/46

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2011-511037 A (TOTAL PETROCHEMICALS RESEARCH FELUY) 07 April 2011 (2011-04-07) examples 1, 4, claim 1, paragraphs [0029], [0045], [0077] | 1-10, 12, 14-15, 17-24 |
| X | JP 2001-026786 A (FINA RESEARCH S. A.) 30 January 2001 (2001-01-30) example 1 | 17, 22-23 |
| X | WO 2012/070605 A1 (ASAHI KASEI CHEMICALS CORPORATION) 31 May 2012 (2012-05-31) examples 12, 23, claim 9, paragraphs [0076]-[0079] | 1-15, 17-24 |
| Y | | 16 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 April 2024** | **16 April 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

| International application No. |
| --- |
| **PCT/JP2024/002643** |

### C. DOCUMENTS CONSIDERED TO BE RELEVANT

| Category* | Citation of document, with indication, where appropriate, of the relevant passages | Relevant to claim No. |
| --- | --- | --- |
| X | WO 2009/031445 A1 (ASAHI KASEI CHEMICALS CORPORATION) 12 March 2009 (2009-03-12)<br>    example 6, table 2, paragraphs [0024], [0025], [0028], [0042] | 1, 3-5, 8-17, 19-24 |
| Y | | 16 |
| A | WO 2009/037992 A1 (ASAHI KASEI CHEMICALS CORPORATION) 26 March 2009 (2009-03-26) | 1-24 |
| A | JP 2016-502487 A (BASF SE) 28 January 2016 (2016-01-28) | 1-24 |

Form PCT/ISA/210 (second sheet) (July 2022)

**INTERNATIONAL SEARCH REPORT**
Information on patent family members

International application No.

**PCT/JP2024/002643**

| Patent document cited in search report | | | Publication date (day/month/year) | Patent family member(s) | | | Publication date (day/month/year) |
|---|---|---|---|---|---|---|---|
| JP | 2011-511037 | A | 07 April 2011 | US examples 1, 4, claim 1, paragraphs [0035], [0051], [0088] | 2011/0098518 | A1 | |
| | | | | WO | 2009/098262 | A1 | |
| | | | | EP | 2090561 | A1 | |
| | | | | KR | 10-2010-0102704 | A | |
| | | | | CN | 101939275 | A | |
| JP | 2001-026786 | A | 30 January 2001 | US example 1 | 2004/0112793 | A1 | |
| | | | | WO | 2000/077122 | A1 | |
| | | | | EP | 1061117 | A1 | |
| WO | 2012/070605 | A1 | 31 May 2012 | US examples 12, 23, claim 9, paragraphs [0130]-[0134] | 2013/0231515 | A1 | |
| | | | | EP | 2644269 | A1 | |
| | | | | CN | 103221130 | A | |
| | | | | KR | 10-2013-0100166 | A | |
| WO | 2009/031445 | A1 | 12 March 2009 | US example 6, table 2, paragraphs [0036], [0037], [0040], [0053] | 2010/0204532 | A1 | |
| | | | | EP | 2184268 | A1 | |
| | | | | CN | 101687726 | A | |
| | | | | KR | 10-2010-0038469 | A | |
| WO | 2009/037992 | A1 | 26 March 2009 | US | 2010/0197986 | A1 | |
| | | | | EP | 2189435 | A1 | |
| | | | | CN | 101772476 | A | |
| | | | | KR | 10-2010-0039415 | A | |
| JP | 2016-502487 | A | 28 January 2016 | EP | 2920112 | A1 | |
| | | | | KR | 10-2015-0086300 | A | |
| | | | | CN | 104918886 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107649173 A
- JP 6229274 B
- JP 5116043 B
- JP 5426983 B

**Non-patent literature cited in the description**

- Adiabatic Fixed-Bed Reactors. Elsevier, 2014, 4
- The Hydrothermal Synthesis of Zeolites. *Chemical Reviews*, 2003, vol. 103, 663-702